# EUROPÄISCHE PATENTSCHRIFT

(11) **EP 3 801 672 B1**
(45) Veröffentlichungstag und Bekanntmachung des Hinweises auf die Patenterteilung: **28.02.2024**
(21) Anmeldenummer: 19728638.8
(22) Anmeldetag: 30.05.2019
(51) Int. Cl.: A61M 60/135, A61M 60/139, A61M 60/216, A61M 60/861, A61M 60/865

(54) **VORRICHTUNG ZUM VERANKERN EINES HERZUNTERSTÜTZUNGSSYSTEMS IN EINEM BLUTGEFÄSS, HERSTELLVERFAHREN ZUM HERSTELLEN EINER VORRICHTUNG UND HERZUNTERSTÜTZUNGSSYSTEM**
APPARATUS FOR ANCHORING A VENTRICULAR ASSIST SYSTEM IN A BLOOD VESSEL, PRODUCTION METHOD FOR PRODUCING AN APPARATUS AND VENTRICULAR ASSIST SYSTEM
DISPOSITIF D'ANCRAGE D'UN SYSTÈME D'ASSISTANCE CARDIAQUE DANS UN VAISSEAU SANGUIN, PROCÉDÉ DE FABRICATION D'UN DISPOSITIF ET SYSTÈME D'ASSISTANCE CARDIAQUE

(30) Priorität: 30.05.2018 DE 102018208555
(43) Veröffentlichungstag der Anmeldung: 14.04.2021
(73) Patentinhaber: Kardion GmbH, 70376 Stuttgart (DE)
(72) Erfinder: BAUMBACH, Hardy, 70376 Stuttgart (DE); SCHUELKE, Armin, 71134 Aidlingen (DE); SCHELLENBERG, Inga, 70191 Stuttgart (DE); MINZENMAY, David, 70569 Stuttgart (DE)
(74) Vertreter: Gauss, Nikolai
(86) Internationale Anmeldenummer: PCT/EP2019/064158
(87) Internationale Veröffentlichungsnummer: WO 2019/229224

(56) Entgegenhaltungen:
- WO-A1-2017/118738
- WO-A2-2008/106103
- CA-A1- 3 000 581
- US-A1- 2013 303 970
- US-A1- 2015 290 372
- US-A1- 2016 271 309

## Beschreibung

Die Erfindung betrifft eine Vorrichtung zum Verankern eines Herzunterstützungssystems in einem Blutgefäß, ein Herstellverfahren zum Herstellen einer derartigen Vorrichtung und ein Herzunterstützungssystem.

Herzunterstützungssysteme zur Langzeittherapie werden typischerweise über eine komplette oder teilweise Eröffnung des Brustbeins implantiert, wobei eine Herzlungenmaschine zum Ermöglichen eines extrakorporalen Blutkreislaufs zum Einsatz kommen kann. Häufig wird hierbei durch das Stanzen eines Lochs in die strukturelle Integrität des Herzmuskelgewebes und der Körperhauptschlagader, der Aorta eingegriffen. Kurzfristige intravaskuläre Herzunterstützungssysteme werden entweder perkutan, also durch die Haut, oder chirurgisch über verschiedene arterielle Zugänge, beispielsweise über die Oberschenkelschlagader, angeliefert. Die endgültige Positionierung der Herzunterstützungssysteme kann intraoperativ visuell unterstützt werden, beispielsweise mittels Ultraschall oder radiologische Durchleuchtung. Die implantierten Herzunterstützungssysteme haben allerdings ein hohes Risiko für eine Dislokation, da keine lokale Fixierung des Herzunterstützungssystems vorhanden ist. Das Herzunterstützungssystem kann also nach der Implantation verrutschen, da es nicht am Implantationsort verankert ist, was zu einer Fehlfunktion der Pumpe oder zu einem Abbruch der Therapie mit dem Herzunterstützungssystem führen kann.

Die CA 3 000 581 A1 zeigt eine Pumpe zum Pumpen von Blut mit in einem distalen Bereich verbundenen Laufrad und ein das Laufrad umgebendes Gehäuse. Das Laufrad und das Gehäuse sind so ausgebildet, dass sie sich nach einer Zwangskompression selbstentfalten.

Die WO 2008/106103 A2 offenbart eine implantierbare Blutpumpe.

Die WO 2017/118738 A1 beschreibt einen Verbinder zur Fluidverbindung zwischen zwei anatomischen Kompartimenten durch mindestens eine anatomische Wand.

Die US 2013/0303970 A1 zeigt eine Katheterpumpe. Die Katheterpumpe kann einen länglichen Katheterkörper mit einem distalen Abschnitt umfassen, der eine expandierbare Kanüle mit einem Einlass und einem Auslass umfasst. Aufgabe der Erfindung ist es, eine Vorrichtung zu schaffen, die es ermöglicht, ein medizinisches System, z.B. ein Herzunterstützungssystem aber auch ein Implantat insbesondere in einem Blutgefäß, z.B. innerhalb der Aorta so anzuordnen, dass dieses in Bezug auf einen Abschnitt des menschlichen oder auch tierischen Körpers, z.B. das Blutgefäß oder die Aorta eine auch über längere Zeiträume hinweg, d.h. Stunden, Tage, Wochen, Monate oder gegebenenfalls auch Jahre eine grundsätzlich gleichbleibende räumliche Lage hat.

Diese Aufgabe wird durch die in Anspruch 1 und 3 angegebene Vorrichtung zum Verankern eines Herzunterstützungssystems in einem Blutgefäß, die in Anspruch 10 angegebene Anordnung und das in Anspruch 11 definierte Herstellungsverfahren für eine Vorrichtung zum Verankern eines Herzunterstützungssystems in einem Blutgefäß gelöst. Vorteilhafte Ausführungsformen der Erfindung sind in den abhängigen Ansprüchen angegeben.

Nachfolgend werden hier die Vorrichtung zum Verankern eines Herzunterstützungssystems in einem Blutgefäß und die Anordnung sowie das Herstellverfahren zum Herstellen einer entsprechenden Vorrichtung vorgestellt. Durch die in den abhängigen Ansprüchen aufgeführten Maßnahmen sind vorteilhafte Weiterbildungen und Verbesserungen der im unabhängigen Anspruch angegebenen Vorrichtung möglich.

Mit dem hier vorgestellten Ansatz kann ein Implantat, beispielsweise ein Herzunterstützungssystem mittels einer Vorrichtung im Blutgefäß, insbesondere innerhalb der Aorta, mit oder ohne räumliche Beziehung zur Aortenklappe, positioniert und verankert werden. Die Vorrichtung kann dazu an einer Herzpumpe fixiert werden. Die Vorrichtung kann vorteilhafterweise zum minimalinvasiven Einführen des Herzunterstützungssystems zusammengefaltet werden, und die Vorrichtung ist so ausgeformt, dass sie sich am Bestimmungsort zum Positionieren und Verankern des Herzunterstützungssystems entfalten kann. Mittels der Vorrichtung kann das Herzunterstützungssystem am Bestimmungsort ausgerichtet und gezielt abgesetzt werden. Die Position des Herzunterstützungssystem bleibt durch die Verankerung mittels der Vorrichtung vorteilhafterweise auch langfristig unverändert, wodurch ein Verrutschen oder eine Dislokation des Herzunterstützungssystems vermieden werden kann.

Es wird eine Vorrichtung zum Verankern eines Herzunterstützungssystems in einem Blutgefäß vorgestellt. Die Vorrichtung kann zum Einführen des Herzunterstützungssystems in das Blutgefäß einen Einführzustand einnehmen. Zudem kann die Vorrichtung einen Verankerungszustand einnehmen, um das Herzunterstützungssystems in dem Blutgefäß zu verankern. Die Vorrichtung umfasst zumindest eine Fixiereinrichtung zum Fixieren der Vorrichtung an das Herzunterstützungssystem, eine Krone und eine Verbindungseinrichtung, die dazu ausgeformt ist, die Krone mit der Fixiereinrichtung zu verbinden. Die Krone ist aus zumindest einem Entfaltungselement ausgeformt. Das Entfaltungselement ist dazu ausgebildet, um sich bei einem Übergang von dem Einführzustand in den Verankerungszustand zu entfalten zum Vergrößern des Durchmessers der Krone, um die Vorrichtung in dem Blutgefäß zu verankern.

Die Vorrichtung kann aus einem biokompatiblen Material ausgeformt sein, um bei einem langfristigen Einsatz der Vorrichtung ein Verwachsen der Vorrichtung mit dem Blutgefäß, beispielsweise der Aorta, zu ermöglichen. Zudem kann die Vorrichtung aus einem elastischen Material realisiert werden, das gleichzeitig eine bestimmte Steifigkeit aufweist, beispielsweise aus Nitinol. Bei dem Herzunterstützungssystem kann es sich beispielsweise um eine Herzpumpe wie ein rechtsventrikuläres Unterstützungssystem, ein linksventrikuläres Unterstützungssystem, ein biventrikuläres Unterstützungssystem oder um eine Gefäß- oder Klappenprothese handeln. Zudem kann die Vorrichtung auch verwendet werden, um eine andere Komponente in einem Blutgefäß zu positionieren und an seinem Bestimmungsort zu verankern, beispielsweise ein anderes vaskuläres oder intrakavitäres Implantat wie beispielsweise ein gastrointestinales, intrathekales, oder intravesikales Implantat. Unter dem Einführzustand kann beispielsweise der Zustand verstanden werden, in dem sich die Vorrichtung zum Einführen oder beim Einführen des Herzunterstützungssystems in das Blutgefäß befinden kann. Die Vorrichtung kann dazu beispielsweise an die Herzpumpe fixiert und so zusammengefaltet sein, dass die Vorrichtung mit dem Herzunterstützungssystem zum minimalinvasiven Einbringen in einen Katheter eingeführt werden kann. Die Vorrichtung und das Herzunterstützungssystem können beispielsweise minimalinvasiv durch die Beinarterie, die arteria fermoralis, eingeführt werden. Im Einführzustand kann die Vorrichtung entsprechend einen Durchmesser aufweisen, der unter dem Durchmesser einer menschlichen Aorta liegt. Unter dem Verankerungszustand kann ein Zustand verstanden werden, in dem die Vorrichtung, nach dem Einführen und Ausrichten am Bestimmungsort zum Vergrößern des Durchmessers der Krone entfaltet ist, um das Herzunterstützungssystem kraftschlüssig am Bestimmungsort zu verankern und auf diese Weise vorteilhafterweise ein Verrutschen oder eine Dislokation des Herzunterstützungssystems zu verhindern. Im Verankerungszustand, also im entfalteten Zustand, kann die Vorrichtung oder eine Komponente der Vorrichtung, beispielsweise die Krone, einen Innendurchmesser aufweisen, der geringfügig größer als der Innendurchmesser des Blutgefäßes ist, in dem es verankert wird, beispielsweise einen gesamten Innendurchmesser im Bereich von 20-30 mm, beispielsweise 23 mm, zum Verankern an oder hinter einer menschlichen Aortenklappe. Zudem kann die Außenkontur zumindest eines Teils der Vorrichtung, beispielsweise der Krone, im Verankerungszustand eine der nicht exakt kreisrunden Aorten-Anatomie einer menschlichen Aorta entsprechende Form aufweisen. Auf diese Weise kann die Vorrichtung im Verankerungszustand über einen radialen Kraftschluss am Bestimmungsort gehalten werden.

Die Fixiereinrichtung kann ausgebildet sein, um die Vorrichtung an einem Implantat, beispielsweise dem Herzunterstützungssystem, zu fixieren. Dazu kann die Fixiereinrichtung zumindest ein Fixierelement zum Bilden einer form-und/oder kraftschlüssigen Verbindung mit einem an dem Implantat angeordneten Gegenfixierelement aufweisen. Die Krone ist ringförmig ausgeformt. Je nach Ausführungsform kann ein einziges Entfaltungselement ringförmig ausgeformt sein oder es können mehrere Entfaltungselemente ringförmig aneinandergereiht sein. Beim Entfalten kann sich das zumindest eine Entfaltungselement ausdehnen. Die Verbindungseinrichtung kann zumindest eine längliche, beispielsweise drahtförmige, Strebe umfassen, die sowohl an der Krone als auch an der Fixiereinrichtung befestigt ist. Die Verbindungseinrichtung kann flexibel ausgeformt sein, um das Vergrößern des Durchmessers der Krone zu ermöglichen.

Einer vorteilhaften Ausführungsform gemäß kann zumindest das Entfaltungselement der Krone der Vorrichtung aus Formgedächtnismaterial ausgeformt sein. Das Entfaltungselement kann beispielsweise aus einem biokompatiblen Formgedächtnispolymer bestehen, oder aus einer biokompatiblen Formgedächtnislegierung, wie beispielsweise Nitinol. Zudem kann auch die gesamte Vorrichtung aus einer Formgedächtnislegierung, beispielsweise aus Nitionol gefertigt werden. Die Verwendung eines Formgedächtnismaterials wie Nitinol ermöglicht aufgrund seiner Formgedächtniseigenschaften eine besonders elegante und einfache Realisierung des Einführzustands und des Entfaltens des Entfaltungselements beim Übergang in den Verankerungszustand. Die Verwendung von Nitinol als Formgedächtnismaterial ist vorteilhaft, da der Nitinolwerkstoff in der Medizin, insbesondere im Bereich der kardiovaskulären Medizin, ein bewährtes Material darstellt, beispielsweise für Herzklappenprothesen, Stents und Gefäßprothesen, aufgrund seiner Biokompatibilität und der Formgedächtniseigenschaft, die es ermöglicht auch komplexe Strukturen auf kleinem Bauraum an den Bestimmungsort zu liefern und abzusetzen, wie die hier vorgestellte Vorrichtung.

Die Vorrichtung kann gemäß einer Ausführungsform zudem eine Bogeneinrichtung mit zumindest einem Füßchen umfassen. Die Bogeneinrichtung ist dazu ausgebildet, um sich bei dem Übergang von dem Einführzustand in den Verankerungszustand zu entfalten zum Positionieren des zumindest einen Füßchens im Blutgefäß. Mittels des Positionierens des zumindest einen Füßchens im Blutgefäß kann die Vorrichtung im Blutgefäß beispielsweise vor dem Verankern ausgerichtet und positioniert werden. Die Bogeneinrichtung kann mit der Krone verbunden sein. Alternativ kann die Bogeneinrichtung eine Bogenfixierungseinrichtung zum Fixieren der Bogeneinrichtung an das Herzunterstützungssystem aufweisen. Die Vorrichtung kann entsprechend einteilig ausgeformt sein, indem die Fixierungseinrichtung, die Krone, die Verbindungseinrichtung und die Bogeneinrichtung miteinander gekoppelt sind, oder die Vorrichtung kann zweiteilig sein, wenn die Bogeneinrichtung mit eigener Bogenfixiereinrichtung an dem Herzunterstützungssystem fixiert ist, und die anderen Komponenten der Vorrichtung miteinander gekoppelt sind. Die einteilige Ausführungsform kann im Hinblick auf das Zusammenfalten der Vorrichtung für den Einführzustand vorteilhaft sein, die zweiteilige Ausführungsform kann je nach Bauweise des Herzunterstützungssystems von Vorteil sein. Wenn das Herzunterstützungssystem beispielsweise eine Pumpe mit Motor umfasst, kann eine zweiteilige Ausführungsform mehr Flexibilität im Hinblick auf den Bauraum des Herzunterstützungssystems ermöglichen, beispielsweise indem die Bogeneinrichtung an einem Ende einer Motor-Kupplung-Pumpeneinheit des Herzunterstützungssystems fixiert werden kann, und der zweite Teil der Vorrichtung an dem anderen Ender der Motor-Kupplung-Pumpeneinheit des Herzunterstützungssystems fixiert werden kann. Mittels der Bogeneinrichtung kann die Vorrichtung vorteilhafterweise durch das Entfalten des zumindest einen Füßchens am Bestimmungsort ausgerichtet und positioniert werden. Das Füßchen kann beispielsweise eine atraumatische Form aufweisen, um das Blutgefäß beim Entfalten und im Verankerungszustand nicht zu verletzen. Vorteilhafterweise kann die Bogeneinrichtung dazu ausgebildet sein, beim Positionieren des zumindest einen Füßchens durch die Ausformung der Bogeneinrichtung und des zumindest einen Füßchens ein Ausrichten der Vorrichtung für den Verankerungszustand zu ermöglichen.

Die Bogeneinrichtung kann gemäß einer weiteren Ausführungsform drei Füßchen aufweisen, insbesondere wobei die Füßchen zum Positionieren der Füßchen in je einer Kuspe einer Herzklappe ausgeformt sind. Diese Ausführungsform ist von Vorteil, um die Vorrichtung und damit das Herzunterstützungssystem besonders exakt positionieren zu können, insbesondere wenn das Herzunterstützungssystem mittels der Vorrichtung beispielsweise hinter der Aortenklappe positioniert und verankert wird. Die drei Füßchen können beispielsweise eine im Hinblick auf eine Erdnuss-Form der Kuspen angepasste Form aufweisen, um so vorteilhafterweise ein besonders vorteilhaftes Verhältnis aus Anlagefläche und Drehsteifigkeit der drei Füßchen zu erreichen.

Für eine kathetergestütze minimalinvasive Implantation des mit der Vorrichtung verbundenen Herzunterstützungssystems ist es von Vorteil, wenn die Vorrichtung gemäß einer Ausführungsform im Einführzustand zylinderförmig ist. Die Vorrichtung kann dazu beispielsweise zusammengefaltet werden. Wenn die Vorrichtung beispielsweise aus Nitinol ausgeformt ist, kann die Vorrichtung aus einem Rohr freigeschnitten werden, anschließend kann eine dem Verankerungszustand entsprechende Form mittels einer Wärmebehandlung eingeprägt werden. Für den Einführzustand kann die Vorrichtung entsprechend der ursprünglich zylinderförmigen Rohrgeometrie zusammengefaltet werden.

Zudem kann es vorteilhaft sein, wenn das Entfaltungselement gemäß einer Ausführungsform im Verankerungszustand eine Schrägstellung aufweist. Unter der Schrägstellung kann ein bestimmter Winkel des Entfaltungselements gegenüber der Längsachse der Vorrichtung verstanden werden. Das Entfaltungselement kann im Verankerungszustand in einem Winkel angestellt sein, um im Verankerungszustand eine kraftschlüssige Verbindung zwischen der Krone und dem Blutgefäß zu unterstützen. Der Winkel kann in Bezug auf die Längsachse der Vorrichtung beispielsweise zwischen 20° und 30° betragen, insbesondere 25°. Durch die Schrägstellung des Entfaltungselements kann die Anpresskraft der Krone an das Blutgefäß vorteilhafterweise erhöht werden.

Die Krone kann gemäß einer Ausführungsform eine Mehrzahl an miteinander gekoppelten Entfaltungselementen aufweisen. Jedes der Entfaltungselemente kann zwei an ihren Enden verbundene Entfaltungsstäbe aufweisen und die zwei Entfaltungsstäbe können im Einführzustand eine geringere Entfernung zueinander aufweisen als im Verankerungszustand. Die Entfaltungselemente können durch eine Verbindung mit einem der Entfaltungsstäbe eines benachbarten Entfaltungselements miteinander gekoppelt sein. Die zwei Entfaltungsstäbe können so miteinander verbunden sein, dass jedes Entfaltungselement im Verankerungszustand der Vorrichtung in axialer Richtung zur Längsachse der Vorrichtung eine Raute mit abgerundeten Ecken ausbildet. Eine solche Ausformung einer Mehrzahl an miteinander gekoppelten rautenförmigen Entfaltungselementen kann einen Standardschnitt eines Gefäß-Stents entsprechen, was beim Herstellen der Vorrichtung vorteilhaft sein kann.

Alternativ kann das Entfaltungselement gemäß einer Ausführungsform eine Mehrzahl an mäanderartig angeordneten Schlaufen aufweisen, wobei die Schlaufen im Einführzustand eine geringere Entfernung zueinander aufweisen als im Verankerungszustand. Diese Ausführungsform bietet ein besonders platzsparendes Zusammenfalten der Vorrichtung für den Einführzustand.

Die Verbindungseinrichtung weist zumindest eine Biegestrebe auf. Die Biegestrebe ist dazu ausgebildet, bei dem Übergang von dem Einführzustand in den Verankerungszustand aufzuklappen, um ein Entfalten der Krone zu ermöglichen. Die Biegestrebe ermöglicht eine besonders elegante und platzsparende Verbindung zwischen der Fixiereinrichtung und der Krone. Die Biegestrebe kann beispielsweise aus einem elastischen Material sein, beispielsweise auch aus Nitinol.

Zudem kann ein erstes Ende der Biegestrebe gemäß einer weiteren Ausführungsform an der Fixiereinrichtung befestigt sein. Ein zweites Ende der Biegestrebe kann an einer Verbindung zwischen zwei benachbarten Entfaltungselementen befestigt sein, oder das zweite Ende der Biegestrebe kann an einem der Fixiereinrichtung abgewandten Ende der Krone befestigt sein. Die Biegestrebe kann also entsprechend der Ausformung des Entfaltungselements der Krone gestaltet werden, um ein effizientes Entfalten der Krone je nach Ausführungsform zu ermöglichen, und um je nach Ausführungsform des Entfaltungselements ein kompaktes Zusammenfalten für den Einführzustand im Hinblick auf die axiale Länge der Vorrichtung zu ermöglichen.

Die Fixiereinrichtung kann gemäß einer Ausführungsform ausgeformt sein, um die Vorrichtung formschlüssig und/oder kraftschlüssig an das Herzunterstützungssystem zu fixieren. Die Vorrichtung kann somit stabil mit dem Herzunterstützungssystem verbunden werden, um beispielsweise während der Implantation oder während der Laufzeit des Herzunterstützungssystems auftretende Kräfte aufzunehmen. Dazu kann die Fixiereinrichtung beispielsweise ein Element zum formschlüssigen Eingreifen aufweisen, oder eine Aussparung zum formschlüssigen Aufnehmen eines Elements, das beispielsweise auf dem Gehäuse des Herzunterstützungssystems aufgetragen ist. Das Element zum formschlüssigen Fixieren kann beispielsweise unterschiedliche Querschnitte aufweisen und das Element oder die entsprechende Aussparung können beispielsweise rund, oval, dreieckig, vieleckig oder sternförmig ausgeformt sein. Sowohl die Fixiereinrichtung als auch das Herzunterstützungssystem, beispielsweise das Gehäuse des Herzunterstützungssystems, können dazu Elemente aufweisen, die ein Einrasten oder Verankern der Fixiereinrichtung mit dem Herzunterstützungssystem ermöglichen. Zusätzlich oder alternativ kann die Fixiereinrichtung beispielsweise eine Bajonettverbindung oder eine Klippverbindung oder einen Haken aufweisen. Zusätzlich oder alternativ kann die Fixiereinrichtung zudem mittels einer stoffschlüssigen Verbindung realisiert werden. Die Fixiereinrichtung kann beispielsweise ausgebildet sein, um eine rotatorische Bewegung zwischen der Vorrichtung und dem Herzunterstützungssystem zu ermöglichen.

Gemäß einer Ausführungsform kann die Vorrichtung zudem eine Hülse aufweisen. Die Hülse kann gegenüber der Krone beweglich sein. Zudem kann die Hülse dazu ausgeformt sein, um im Einführzustand zumindest die Krone zu umschließen und die Krone zum Einleiten des Übergangs in den Verankerungszustand freizugeben. Wenn die Vorrichtung gemäß einer Ausführungsform die Bogeneinrichtung umfasst, kann die Hülse auch gegenüber der Bogeneinrichtung beweglich sein und zudem dazu ausgeformt sein im Einführzustand auch die Bogeneinrichtung zu umschließen und die Bogeneinrichtung zum Einleiten des Übergangs in den Verankerungszustand freizugeben. Die Hülse kann auch dazu ausgeformt sein, alle anderen Komponenten der Vorrichtung zu umschließen und wieder freizugeben. Die Hülse kann beispielsweise zylinderförmig ausgeformt und so ausgeführt sein, dass die Vorrichtung mit der Hülse im Einführzustand in einen handelsüblichen Katheter eingeführt werden kann. Die Hülse kann vorteilhafterweise beispielsweise verwendet werden, um die anderen Komponenten der Vorrichtung im zusammengefalteten Zustand der Vorrichtung niederzuhalten und dadurch im Einführzustand zusätzlich zu stabilisieren, beispielsweise auch, wenn Komponenten der Vorrichtung oder die gesamte Vorrichtung aus einem Formgedächtnismaterial ausgeformt sind. Zudem kann die Hülse beim Übergang vom Einführzustand in den Verankerungszustand schrittweise entfernt werden, beispielsweise über einen gesteuerten Mechanismus, der beispielsweise elektrisch gesteuert werden kann, oder durch ein manuelles Zurückziehen der Hülse. Auf diese Weise kann die Vorrichtung beispielsweise schrittweise entfaltet werden, um die Vorrichtung vorteilhafterweise kontrolliert zu entfalten und vor dem Verankern zu positionieren oder die Hülse kann wieder nach vorne verschoben werden, um die Vorrichtung anders auszurichten oder das Positionieren der Vorrichtung zu korrigieren.

Mit diesem Ansatz wird zudem ein Verfahren (nicht beansprucht) zum Betreiben der Vorrichtung vorgestellt. Das Verfahren weist zumindest einen Schritt des Entfaltens auf. Im Schritt des Entfaltens wird das Entfaltungselement der Krone der Vorrichtung bei dem Übergang von dem Einführzustand in den Verankerungszustand zum Vergrößern des Durchmessers der Krone entfaltet. Der Schritt des Entfaltens kann beispielsweise auch durchgeführt werden, um den Durchmesser der Krone zum Verankern der Vorrichtung in dem Blutgefäß zu vergrößern. Das Verfahren kann sowohl ausgeführt werden, wenn die Vorrichtung innerhalb eines Blutgefäßes angeordnet ist, als auch wenn sich die Vorrichtung außerhalb eines Blutgefäßes befindet, beispielsweise um durch ein Vergrößern des Durchmessers der Krone die Vorrichtung mit einer Komponente eines Implantats zu verbinden.

Es wird zudem ein Herstellverfahren zum Herstellen einer Vorrichtung zum Verankern eines Herzunterstützungssystems in einem Blutgefäß vorgestellt. Die Vorrichtung kann einen Einführzustand zum Einführen des Herzunterstützungssystems in das Blutgefäß einnehmen, und ferner kann die Vorrichtung einen Verankerungszustand einnehmen, um das Herzunterstützungssystems in dem Blutgefäß zu verankern. Das Herstellverfahren weist zumindest einen Schritt des Bereitstellens, einen Schritt des Ausformens und einen Schritt des Wärmebehandelns auf. Im Schritt des Bereitstellens wird ein Halbzeug aus einem Formgedächtnismaterial bereitgestellt. Im Schritt des Ausformens wird eine Fixiereinrichtung zum Fixieren der Vorrichtung an das Herzunterstützungssystem ausgeformt. Zudem wird im Schritt des Ausformens eine Krone aus zumindest einem Entfaltungselement ausgeformt, wobei das Entfaltungselement dazu ausgebildet ist, um sich bei einem Übergang von dem Einführzustand in den Verankerungszustand zu entfalten zum Vergrößern des Durchmessers der Krone, um die Vorrichtung in dem Blutgefäß zu verankern. Ferner wird im Schritt des Ausformens eine Verbindungseinrichtung dazu ausgeformt, die Krone mit der Anbindungseinrichtung zu verbinden. Die Fixierungseinrichtung, die Krone und die Verbindungseinrichtung werden aus dem Halbzeug ausgeformt. Im Schritt des Wärmebehandelns werden Fixierungseinrichtung, die Krone und die Verbindungseinrichtung wärmebehandelt, um die Form des Verankerungszustands einzuprägen.

Es wird außerdem ein Herzunterstützungssystem mit einer Vorrichtung gemäß einer Ausführungsform vorgestellt. Die Vorrichtung kann beispielsweise an dem Herzunterstützungssystem fixiert sein. Das Herzunterstützungssystem kann beispielsweise eine Herzpumpe mit einer Motor-Kupplung-Pumpeneinheit umfassen. Zudem kann das Herzunterstützungssystem ein Gehäuse aufweisen, das ausgeformt ist, um mit der Vorrichtung, beispielsweise mittels der Fixiereinrichtung der Vorrichtung, formschlüssig und/oder kraftschlüssig verbunden zu sein. Vorteilhafterweise können gemäß dieser Ausführungsform die Größe des Herzunterstützungssystem und/oder die Abmessungen der Vorrichtung patientenspezifisch ausgewählt oder verändert werden.

Ausführungsbeispiele des hier vorgestellten Ansatzes sind in den Zeichnungen dargestellt und in der nachfolgenden Beschreibung näher erläutert. Es zeigt:
- Fig. 1: eine schematische Darstellung einer Vorrichtung zum Verankern eines Herzunterstützungssystems in einem Blutgefäß gemäß einem Ausführungsbeispiel;
- Fig. 2: eine schematische Darstellung eines Teils eines Herzunterstützungssystems mit einer Vorrichtung zum Verankern eines Herzunterstützungssystems in einem Blutgefäß gemäß einem Ausführungsbeispiel;
- Fig. 3: eine schematische Darstellung eines Herzunterstützungssystems mit einer Vorrichtung zum Verankern eines Herzunterstützungssystems in einem Blutgefäß gemäß einem Ausführungsbeispiel;
- Fig. 4 und 5: eine schematische Darstellung eines Einführzustandes einer Vorrichtung zum Verankern eines Herzunterstützungssystems in einem Blutgefäß gemäß einem Ausführungsbeispiel;
- Fig. 6: eine schematische Darstellung einer Vorrichtung zum Verankern eines Herzunterstützungssystems in einem Blutgefäß mit einer Bogeneinrichtung gemäß einem Ausführungsbeispiel;
- Fig. 7: eine schematische Darstellung eines Füßchens einer Vorrichtung zum Verankern eines Herzunterstützungssystems in einem Blutgefäß gemäß einem Ausführungsbeispiel;
- Fig. 8: eine schematische Darstellung eines Teils einer Krone einer Vorrichtung zum Verankern eines Herzunterstützungssystems in einem Blutgefäß gemäß einem Ausführungsbeispiel;
- Fig. 9 und 10: eine schematische Darstellung einer Fixiereinrichtung einer Vorrichtung zum Verankern eines Herzunterstützungssystems in einem Blutgefäß gemäß einem Ausführungsbeispiel;
- Fig. 11a bis 11j: eine schematische Darstellung einer Ausformung einer formschlüssigen Fixierung einer Vorrichtung zum Verankern eines Herzunterstützungssystems in einem Blutgefäß gemäß einem Ausführungsbeispiel;
- Fig. 12: eine schematische Darstellung einer kraftschlüssigen Fixierung einer Vorrichtung zum Verankern eines Herzunterstützungssystems in einem Blutgefäß an ein Herzunterstützungssystem gemäß einem Ausführungsbeispiel;
- Fig. 13 bis 17: eine schematische Darstellung eines Teils einer Fixiereinrichtung einer Vorrichtung zum Verankern eines Herzunterstützungssystems in einem Blutgefäß gemäß einem Ausführungsbeispiel;
- Fig. 18a bis 18c: eine schematische Darstellung einer Vorrichtung zum Verankern eines Herzunterstützungssystems in einem Blutgefäß gemäß einem Ausführungsbeispiel;
- Fig. 19a bis 19c: eine schematische Darstellung einer Vorrichtung zum Verankern eines Herzunterstützungssystems in einem Blutgefäß gemäß einem Ausführungsbeispiel;
- Fig. 20: ein Ablaufdiagramm eines Verfahrens zum Betreiben einer Vorrichtung zum Verankern eines Herzunterstützungssystems in einem Blutgefäß gemäß einem Ausführungsbeispiel; und
- Fig. 21: ein Ablaufdiagramm eines Herstellverfahrens zum Herstellen einer Vorrichtung zum Verankern eines Herzunterstützungssystems in einem Blutgefäß gemäß einem Ausführungsbeispiel.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

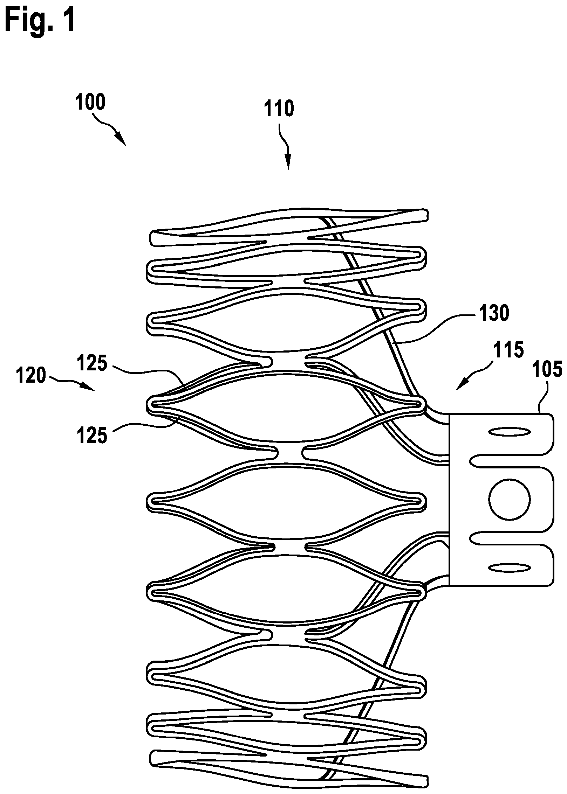

Fig. 1 zeigt eine schematische Darstellung einer Vorrichtung 100 zum Verankern eines Herzunterstützungssystems in einem Blutgefäß gemäß einem Ausführungsbeispiel. Auch wenn die Vorrichtung 100 hier und im Folgenden im Zusammenhang mit einem Herzunterstützungssystem beschrieben ist, so kann die Vorrichtung 100 auch zum Verankern anderer Implantate in einem Blutgefäß eingesetzt werden.

Gezeigt ist eine Seitenansicht der entfalteten Vorrichtung 100. Die Vorrichtung 100 kann einen Einführzustand zum Einführen des Herzunterstützungssystems in das Blutgefäß einnehmen. Zudem kann die Vorrichtung 100 den in Fig. 1 gezeigten Verankerungszustand einnehmen, um das Herzunterstützungssystems in dem Blutgefäß zu verankern.

Die Vorrichtung 100 weist zumindest eine Fixiereinrichtung 105 zum Fixieren der Vorrichtung 100 an das Herzunterstützungssystem, eine Krone 110 und eine Verbindungseinrichtung 115 auf. Die Krone 110 ist aus zumindest einem Entfaltungselement 120 ausgeformt. Das Entfaltungselement 120 ist dazu ausgebildet, um sich bei einem Übergang von dem Einführzustand in den Verankerungszustand zu entfalten zum Vergrößern des Durchmessers der Krone 110, um die Vorrichtung 100 in dem Blutgefäß zu verankern. Gemäß dem gezeigten Ausführungsbeispiel umfasst die Krone 110 eine Mehrzahl von Entfaltungselementen 120. Die Verbindungseinrichtung 115 ist dazu ausgeformt, die Krone 110 mit der Fixiereinrichtung 105 zu verbinden.

Vorteilhafterweise kann mittels der Vorrichtung 100 ein Verrutschen oder Bewegen des Herzunterstützungssystems verhindert werden, da mittels der Krone 110 eine Verankerung am Implantationsort stattfinden kann. Zudem ist mittels der Vorrichtung 100 eine definierte Positionierung des Herzunterstützungssystems, im Folgenden auch genannt, möglich. Die Vorrichtung kann zumindest teilweise aus einem Formgedächtnismaterial wie Nitinol ausgeformt sein. In der hier gezeigten Figur 1 ist die Vorrichtung 100 als Nitinolrahmen zur Verankerung und Positionierung eines Herzunterstützungssystems in einem Blutgefäß ausgeformt. Die Vorrichtung 100 kann sowohl ein Design für den Langzeiteinsatz aufweisen, als auch für kurzzeitige Implantationen ein rückholbares Design aufweisen, beispielsweise durch ein gesteuertes Überführen der implantierten und verankerten Vorrichtung 100 vom Verankerungszustand in den Einführzustand.

Die die Krone 110 und die Fixiereinrichtung 105 der Vorrichtung 100 sind entlang einer Längsachse der Vorrichtung 100 erstreckt, die der Achse eines Katheters entsprechen kann, in dem das Herzunterstützungssystem minimalinvasiv durch die Beinarterie eingeführt wird.

In der hier gezeigten Darstellung ist das Entfaltungselement 120 und damit die Krone 110 entsprechend dem Verankerungszustand entfaltet. Das Entfaltungselement 120 kann gemäß dem gezeigten Ausführungsbeispiel im Verankerungszustand eine Schrägstellung in Bezug auf die Längsachse der Vorrichtung 100 aufweisen. Dazu kann das Entfaltungselement 120 in einen bestimmten Winkel angestellt sein, beispielsweise in einem Winkel von 25°, um eine erhöhte Anpresskraft der Krone 110 zum kraftschlüssigen Verankern der Vorrichtung 100 in dem Blutgefäß zu erzeugen.

Die Krone 110 kann wie in dem hier gezeigten Ausführungsbeispiel eine Mehrzahl an miteinander gekoppelten Entfaltungselementen 120 aufweisen, wobei jedes der Entfaltungselemente 120 zwei an ihren Enden verbundene Entfaltungsstäbe 125 aufweist. Mittige Abschnitte der zwei Entfaltungsstäbe 125 jedes Entfaltungselements 120 weisen im Einführzustand eine geringere Entfernung zueinander auf als im Verankerungszustand. Die mittigen Abschnitte aller Entfaltungselemente 120 sind gemäß diesem Ausführungsbeispiel auf einer Kreisbahn angeordnet. Im entfalteten Zustand kann jedes Entfaltungselement 120 eine Rauten-Form mit abgerundeten Ecken aufweisen, wobei die Rauten-Form durch die Entfernung der zwei an ihren Enden verbundenen Entfaltungsstäbe 125 ausgebildet wird. Die miteinander gekoppelten Entfaltungselemente 120 bilden einen gitterförmigen Ring. Die Entfaltungselemente 120 können identisch ausgeformt sein.

Die Verbindungseinrichtung 115 weist optional zumindest eine Biegestrebe 130 auf. Die Biegestrebe 130 ist dazu ausgebildet, bei dem Übergang von dem Einführzustand in den Verankerungszustand aufzuklappen, um ein Entfalten der Krone 110 zu ermöglichen. Die Verbindungseinrichtung 115 kann auch mehrere Biegestreben 130, beispielsweise um ein besonders gleichmäßiges Entfalten des Entfaltungselements 120 und damit der Krone 110 zu ermöglichen. In dem hier gezeigten Ausführungsbeispiel weist die Vorrichtung 100 beispielhaft vier gleich beabstandete Biegestreben 130 auf.

Gemäß einem Ausführungsbeispiel ist ein erstes Ende der Biegestrebe 130 an der Fixiereinrichtung 105 befestigt ist und ein zweites Ende der Biegestrebe 130 ist wie hier gezeigt an einer Verbindung zwischen zwei benachbarten Entfaltungselementen 120 befestigt. Alternativ kann das zweite Ende der Biegestrebe 130 an einem der Fixiereinrichtung 105 abgewandten Ende der Krone 110 befestigt sein.

Die Fixiereinrichtung 105 ist gemäß einem Ausführungsbeispiel dazu ausgeformt, die Vorrichtung 100 formschlüssig und/oder kraftschlüssig an das Herzunterstützungssystem zu fixieren. Die Fixiereinrichtung 105 kann dazu beispielsweise wie hier gezeigt eine Aussparung zum formschlüssigen Aufnehmen eines auftragenden Elements aufweisen, wobei das entsprechende Element in einer Komponente des zu fixierenden Herzunterstützungssystems ausgeformt sein kann.

Gemäß einem Ausführungsbeispiel ist die Fixiereinrichtung 105 als ein Ring ausgeformt, der eine Mehrzahl von Aussparungen aufweist. Im entfalteten Zustand der Krone 110 weist die ringförmige Krone 110 einen größeren Durchmesser als die als Ring ausgeformte Fixiereinrichtung 105 auf.

Die Krone 110 und die Fixiereinrichtung 105 weisen gemäß einem Ausführungsbeispiel keine oder nur eine geringe Überlappung auf.

Eine Längsachse der Vorrichtung 100 verläuft gemäß einem Ausführungsbeispiel mittig durch die Krone 110 und die Fixiereinrichtung 105. Gemäß einem Ausführungsbeispiel weisen die Entfaltungselemente 120 im entfalteten Zustand eine Schrägstellung auf, wobei der Fixiereinrichtung 105 zugewandte Enden der Entfaltungselemente 120 einen größeren Abstand zur Längsachse der Vorrichtung 100 als der Fixiereinrichtung 105 abgewandte Enden der Entfaltungselemente 120 aufweisen.

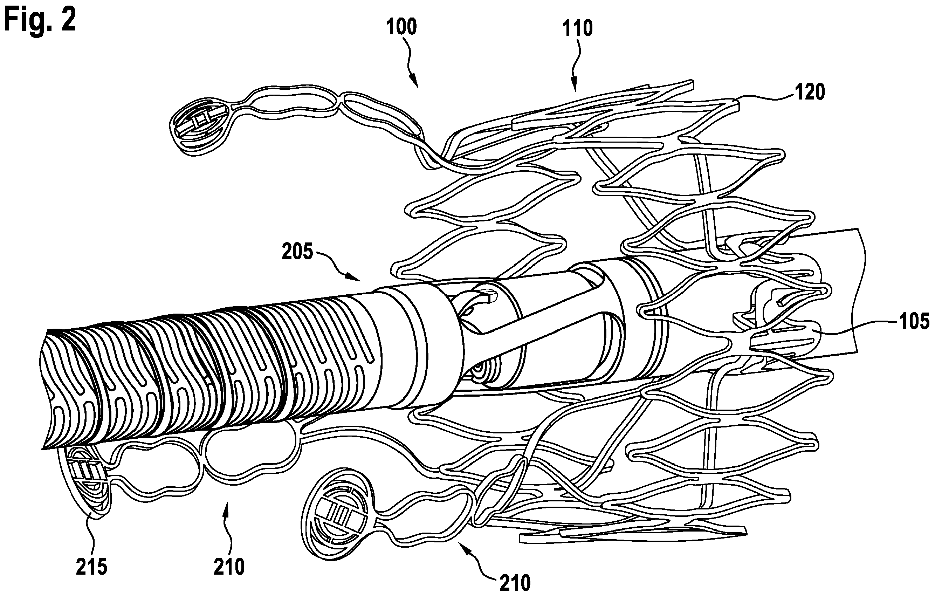

Fig. 2 zeigt eine schematische Darstellung eines Teils eines Herzunterstützungssystems 205 mit einer Vorrichtung 100 zum Verankern des Herzunterstützungssystems 205 in einem Blutgefäß gemäß einem Ausführungsbeispiel. Als Teils des Herzunterstützungssystems 205 ist beispielhaft ein Abschnitt einer Herzpumpe mit rohrförmigen Komponenten gezeigt, die einen Motor und ein Laufrad der Herzpumpe umfassen. In der hier gezeigten Aufsicht ist die Vorrichtung 100 an einem Gehäuseabschnitt des Herzunterstützungssystems 205 fixiert.

Das Entfaltungselement 120 kann gemäß einem Ausführungsbeispiel aus einem Formgedächtnismaterial ausgeformt sein. Optional können auch mehrere Komponenten oder die gesamte Vorrichtung 100 aus einem Formgedächtnismaterial ausgeformt sein, beispielsweise wie hier gezeigt aus Nitinol.

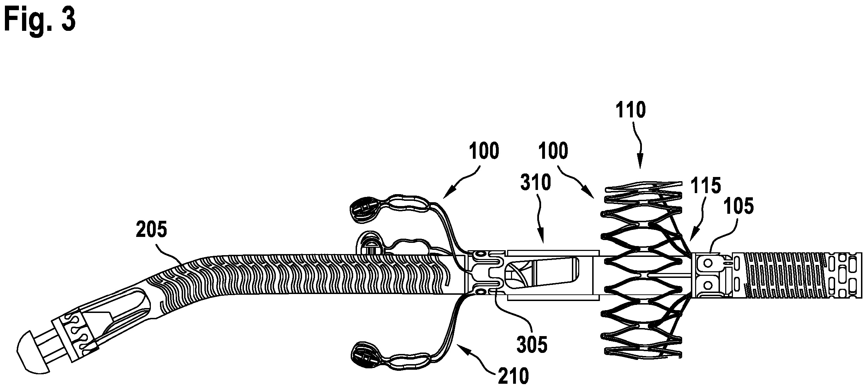

Gemäß dem hier gezeigten Ausführungsbeispiel umfasst die Vorrichtung 100 eine Bogeneinrichtung 210 mit zumindest einem Füßchen 215. Die Bogeneinrichtung 210 ist dazu ausgebildet, um sich bei dem Übergang von dem Einführzustand in den Verankerungszustand zu entfalten und so ein Positionieren des zumindest einen Füßchens 215 im Blutgefäß zu ermöglichen. Die Bogeneinrichtung 210 ist hier mit der Krone 110 verbunden. Alternativ kann die Bogeneinrichtung 210 auch eine Bogenfixierungseinrichtung zum Fixieren der Bogeneinrichtung 210 an das Herzunterstützungssystem 205 aufweisen, dies ist in der folgenden Figur 3 gezeigt.

Die Bogeneinrichtung 210 kann, wie hier gezeigt, drei Füßchen 215 aufweisen. Insbesondere können die Füßchen 215 zum Positionieren der Füßchen 215 in je einer Kuspe einer Herzklappe ausgeformt sein, beispielsweise wenn das durch die Vorrichtung 100 fixierte Herzunterstützungssystem 205 innerhalb einer menschlichen Aorta direkt hinter einer Aortenklappe positioniert und verankert wird.

Die Fixiereinrichtung 105 kann die Vorrichtung 100 mittels eines formschlüssigen, kraftschlüssigen oder stoffschlüssigen Anbindungsmechanismus an das Herzunterstützungssystem fixieren. Dazu kann das Herzunterstützungssystem 205 beispielsweise wie hier gezeigt ein Verbindungselement zum formschlüssigen Eingreifen der Fixiereinrichtung 105 aufweisen, und die Fixiereinrichtung 105 kann eine entsprechenden Materialaussparung zum Eingreifen aufweisen, oder ein entsprechend ausgeformtes Verbindungselement zum Eingreifen, wie die hier gezeigte Klippverbindung. Auf diese Weise kann das Herzunterstützungssystem 205 an die Vorrichtung 100 fixiert werden, zum Verankern des Herzunterstützungssystems 205 in dem Blutgefäß, beispielsweise in der Aorta. Die Krone 110 sorgt dafür, dass das Herzunterstützungssystems 205 durch einen radialen Kraftschluss der Vorrichtung mit einem Wandabschnitt der Aorta in der Aorta gehalten wird.

Fig. 3 zeigt eine schematische Darstellung eines Herzunterstützungssystems 205 mit einer Vorrichtung 100 zum Verankern eines Herzunterstützungssystems 205 in einem Blutgefäß gemäß einem Ausführungsbeispiel. Gezeigt ist eine Seitenansicht des Herzunterstützungssystems 205 mit der Vorrichtung 100, wobei die Vorrichtung 100 zweiteilig ausgeführt ist. Ein Teil der Vorrichtung 100 umfasst dabei die mittels der Verbindungseinrichtung 115 mit der Fixiereinrichtung 105 verbundene Krone 110, und ein anderer Teil der Vorrichtung 100 besteht aus der Bogeneinrichtung 210. Die Vorrichtung 100 befindet sich in einem dem Verankerungszustand entsprechenden entfaltetem Zustand.

Die Bogeneinrichtung 210 weist gemäß dem hier gezeigten Ausführungsbeispiel eine Bogenfixierungseinrichtung 305 zum Fixieren der Bogeneinrichtung 210 an das Herzunterstützungssystem 205 auf und ist nicht mit der Krone 110 verbunden. Die Bogenfixierungseinrichtung 305 kann die Bogeneinrichtung 210 wie hier beispielhaft gezeigt mittels einer formschlüssigen und kraftschlüssigen Verbindung an das Herzunterstützungssystem 205 fixieren.

Das Einbringen des Herzunterstützungssystems 205 wie der hier beispielhaft gezeigten Herzpumpe kann bevorzugt minimalinvasiv durch eine menschliche Beinarterie, die Arteria femoralis, erfolgen. Für die Komponenten der Vorrichtung 100 kann entsprechend ein begrenzter Einführdurchmesser zur Verfügung stehen, der im maximalen Durchmesser der Arteria femoralis im Bereich der Implantationsstelle oder anderer Arterien im Verlauf (beispielsweise der arteria iliaca etc.) oder aber z. B. der Gewundenheit oder den Verkalkungsgraden der arteriellen Strombahn begründet ist. Das Herzunterstützungssystem 205 sowie die Vorrichtung 100, die auf diesem Weg in den Körper gebracht werden können, können somit im Durchmesser sowie in der Baulänge limitiert sein. Bei einer Herzpumpe als Herzunterstützungssystem 205 kann eine hohe Drehzahl des Motors sowie des Pumpenrades der Herzpumpe eingestellt werden, um trotzdem eine signifikante Unterstützung des Herzens zu erzielen. Die Miniaturisierung dieser Komponenten der Herzpumpe in Kombination mit der hohen Drehzahl kann zur Folge haben, dass der Wirkungsgrad des Elektromotors sinkt, und dass durch die kleine wärmeabführende Oberfläche die Oberflächentemperatur ansteigt. Wenn die Vorrichtung 100 eine Bogeneinrichtung 120 aufweist und gemäß einer Ausführungsform einteilig ausgeführt ist, wie in der vorhergehenden Figur 2 gezeigt, kann die Vorrichtung im Einführzustand eng an den rohrförmigen Komponenten des Herzunterstützungssystems 205 anliegen, um den Durchmesser möglichst klein zu halten. Die zusammengefaltete einteilige Vorrichtung 100 kann sich dabei über die gesamte Länge der Motor-Kupplung-Pumpeneinheit des Herzunterstützungssystems 205 erstrecken. In diesem Fall ist eine zweiteilig ausgeführte Vorrichtung wie das in der vorliegenden Figur 3 gezeigte Ausführungsbeispiel vorteilhaft: Durch eine zweiteilige Ausführung der Vorrichtung 100 und somit auch zweiteilige Verankerung des Herzunterstützungssystems 205 im Körper kann sich im Bereich des Motors und Laufrades der Herzpumpe radial Bauraum einsparen lassen, beispielsweise in dem hier gekennzeichneten Bereich 310 des Herzunterstützungssystems 205. Ein derart gewonnener Bauraum im Bereich 310 kann beispielsweise genutzt werden, um den Pumpenrotor des Herzunterstützungssystems 205 zu vergrößern und damit die Drehzahl abzusenken, um den Wirkungsgrad zu verbessern. Oder es kann im Bereich 310 der Durchmesser der Kupplung zwischen Motor und Pumpenrotor des Herzunterstützungssystems 205 vergrößert werden, um ein höheres Drehmoment übertragen zu können, oder um eine Baulänge der Kupplung des Herzunterstützungssystems 205 einzusparen. Zudem kann der Bereich 310 genutzt werden, um Strömungsleitgeometrien, beispielsweise Leitschaufeln, am Gehäuse des Herzunterstützungssystems 205 anzubringen, um dadurch den Wirkungsgrad zu erhöhen und die Blutströmung zu beruhigen.

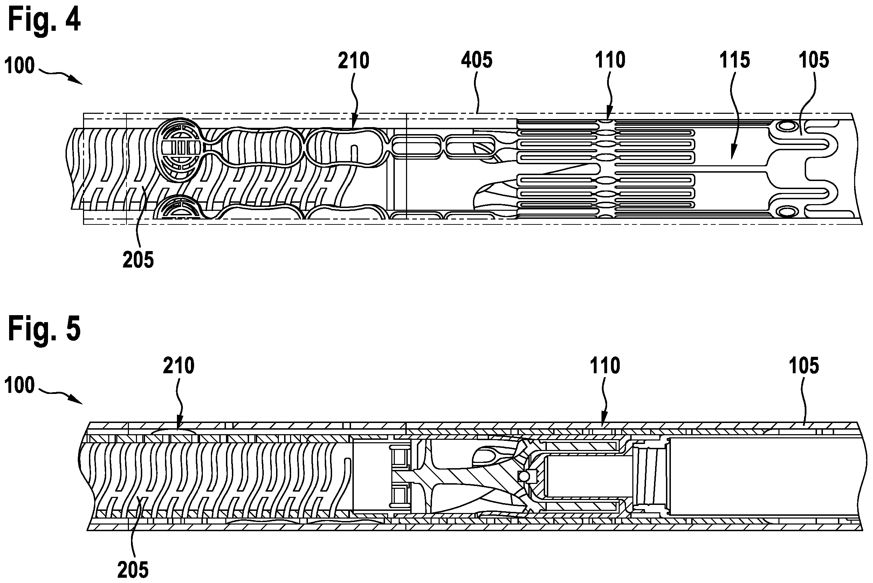

Fig. 4 zeigt eine schematische Darstellung eines Einführzustandes einer Vorrichtung 100 zum Verankern eines Herzunterstützungssystems 205 in einem Blutgefäß gemäß einem Ausführungsbeispiel, gezeigt in einer Seitenansicht. Die Vorrichtung 100 umfasst hier eine Hülse 405, in die das Herzunterstützungssystem 205 eingeführt ist. Das Herzunterstützungssystem 205 ist zudem mittels der Fixiereinrichtung 105 mit der Vorrichtung 100 verbunden, und die Verbindungseinrichtung 115 verbindet die Krone 110 mit der Fixiereinrichtung 105. Die Krone 110 ist mit der Bogeneinrichtung 210 verbunden.

Gemäß dem hier gezeigten Ausführungsbeispiel ist die Vorrichtung 100 im Einführzustand zylinderförmig. Dazu sind die Verbindungseinrichtung 115, die Krone 110 und die Bogeneinrichtung 210 zylinderförmig zusammengefaltet, alle Komponenten der Vorrichtung liegen an das Herzunterstützungssystem 205 und/oder einer je anderen Komponente der Vorrichtung 100 an.

Das Entfaltungselement, aus dem die Krone 110 ausgeformt ist, ist optional aus einem Formgedächtnismaterial, beispielsweise Nitinol, ausgeformt. Zudem können auch weitere Komponenten der Vorrichtung, wie beispielsweise die Bogeneinrichtung 210 sowie die Verbindungseinrichtung 115 aus einem Formgedächtnismaterial bestehen. Wenn beispielsweise die Krone 110, die Verbindungseinrichtung 115 und die Bogeneinrichtung 210 aus einem Nitinolrohr freigeschnitten werden, können diese Komponenten, aufgrund der pseudoelastischen Eigenschaften des Formgedächtnismaterials, für den Implantationsvorgang auf ihre ursprüngliche zylindrische Form, die Rohrgeometrie, zusammengefaltet werden. Auf diese Weise kann durch den geringen Bauraum eine kathetergestützte minimalinvasive Implantation ermöglicht werden. Wenn die Vorrichtung 100 wie hier eine Hülse 405 aufweist, können die Verbindungseinrichtung 115, die Krone 110 und die Bogeneinrichtung 210 von der Hülse 405 niedergehalten und dadurch zusätzlich oder alternativ am Entfalten gehindert werden. In der hier gezeigten Darstellung werden entsprechend die genannten Komponenten der Vorrichtung 100, die Krone 110, die Verbindungseinrichtung 115 und die Bogeneinrichtung 210, im zusammengefalteten Zustand innerhalb der Hülse 405 gezeigt.

Wenn die Vorrichtung 100 gemäß einem Ausführungsbeispiel die Hülse 405 aufweist, ist die Hülse 405 gegenüber der Krone 110 beweglich. Zudem ist die Hülse 405 dazu ausgeformt, um im Einführzustand zumindest die Krone 110 zu umschließen und zumindest die Krone 110 zum Einleiten des Übergangs in den Verankerungszustand freizugeben. Die Hülse 405 kann somit verwendet werden, um insbesondere die Krone 110 und die Bogeneinrichtung 210 während des Implantationsvorgangs niederzuhalten, damit sich diese Komponenten nicht entfalten und somit nicht aufstellen. Dazu wird die Hülse 405, auch Release Sheath genannt, beim Laden des mit der Fixiereinrichtung 105 der Vorrichtung 100 verbundenen Herzunterstützungssystems 205 in einen Katheter über die anderen Komponenten der Vorrichtung 100 geschoben. Sobald die endgültige Position des Herzunterstützungssystems 205, beispielsweise vor der Herzklappe, erreicht ist, wird die Hülse 405 zurückgezogen, sodass sich die Krone 110 und die Bogeneinrichtung 210 entfalten können. Bis dahin kann der Operateur sowohl die axiale Position, als auch die rotatorische Ausrichtung, die Position des Ansaugschlauchs des Herzunterstützungssystems 205 im Ventrikel reversibel bestimmen. Weiterhin kann die Hülse 405 schrittweise zurückgezogen werden, sodass es beim Übergang vom Einführzustand in den Verankerungszustand zu einer langsamen, stufenweisen Freisetzung der Bogeneinrichtung 210 und der Krone 110 kommt.

Fig. 5 zeigt eine schematische Darstellung eines Einführzustandes einer Vorrichtung 100 zum Verankern eines Herzunterstützungssystems 205 in einem Blutgefäß gemäß einem Ausführungsbeispiel. Es ist eine Schnittansicht der in der vorhergehenden Fig. 4 dargestellten Seitenansicht gezeigt. Entsprechend sind die Bogeneinrichtung 210 und die Krone 110 im geladenen Zustand und werden zusammen mit der Verbindungseinrichtung und der Fixiereinrichtung 105 von der Hülse 405 umschlossen, in die das an die Vorrichtung 100 fixierte Herzunterstützungssystem 205 eingeführt ist. Durch den hier gezeigten geladenen Zustand sind die Komponenten der Vorrichtung 100 innerhalb der Hülse 405 nur schwer von dem Herzunterstützungssystem 205 unterscheidbar, da sie eng an das Herzunterstützungssystem 205 anliegen. Dies illustriert die Möglichkeit, die Vorrichtung 100 so zusammenzufalten, dass das an die Vorrichtung 100 fixierte Herzunterstützungssystems 205 minimalinvasiv eingeführt und implantiert werden kann.

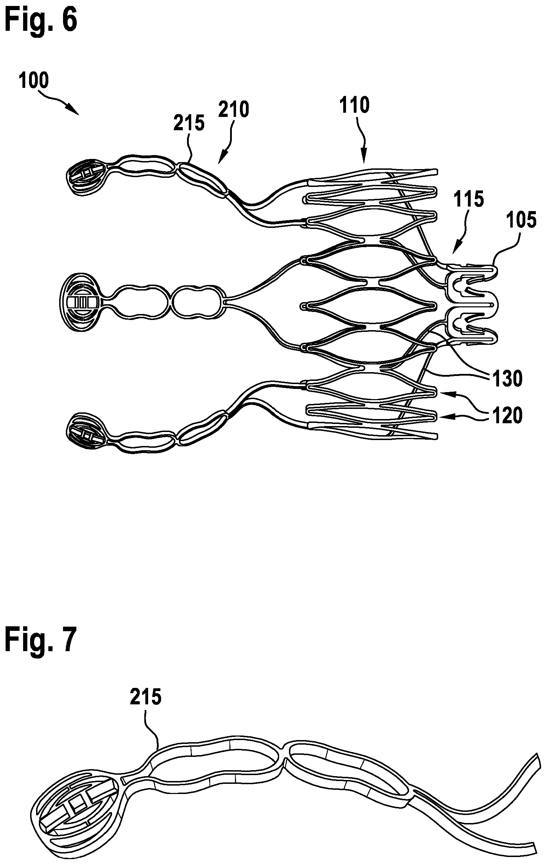

Fig. 6 zeigt eine schematische Darstellung einer Vorrichtung 100 zum Verankern eines Herzunterstützungssystems in einem Blutgefäß mit einer Bogeneinrichtung 210 gemäß einem Ausführungsbeispiel. Es ist eine Seitenansicht der Vorrichtung 100 im entfalteten Zustand in einteiliger Ausführung gezeigt. Die Fixiereinrichtung 105 weist die Ausformung und Aussparungen zum formschlüssigen Eingreifen in ein auf dem Herzunterstützungssystem aufgetragenes Element mittels einer Klippverbindung auf. Die Verbindungseinrichtung 115 besteht hier aus vier je an einem Ende mit der Fixiereinrichtung 105 verbundenen Biegestreben 130. Die Krone 110 besteht aus einer Mehrzahl an miteinander gekoppelten Entfaltungselementen 120, deren Entfaltungsstäbe jeweils Rauten ausbilden. Das je andere Ende der vier Biegestreben 130 der Verbindungseinrichtung 115 ist mit einer Verbindung zweier benachbarter Entfaltungselemente 120 verbunden. Die Bogeneinrichtung 210 ist am der Fixiereinrichtung 105 gegenüberliegenden Ende der Krone 110 mit der Krone 110 verbunden. Die Bogeneinrichtung 210 weist hier drei Füßchen 215 auf. Die Füßchen 215 sind je in einem ihrer Abschnitte angrenzend an die Verbindung mit der Krone 110 zunächst talförmig in Richtung der Längsachse der Vorrichtung 100 gebogen, und bilden anschließend in einem Abschnitt länger als die Hälfte der Länge jedes Füßchen 215 einen halbkreisförmigen Bogen aus.

Die hier gezeigte Krone 110 kann beispielsweise im zum Verankern des Herzunterstützungssystems entfalteten Zustand einen Außendurchmesser aufweisen, der etwas größer als eine menschliche Aorta ist, um einen gleichmäßigen Kraftschluss mit dem Blutgefäß in Form der Aorta zu ermöglichen, der Außendurchmesser kann in diesem Fall beispielsweise 20- 30 mm, betragen.

Die Anpresskraft der Krone 110 und damit auch der Vorrichtung 100wird durch die Stege in Form der Entfaltungselemente 120 erzeugt, die mit einem Winkel von 20°-30°, insbesondere ca. 25°, angestellt sind. Die Krone 110 weist vorteilhafterweise eine konische Form mit 5°-10° Winkel auf, dies kann beim Release-Vorgang, also beim Übergang vom Einführzustand in den Verankerungszustand, eine durchgehende tangentiale Verbindung zum Release-Sheath in Form der Hülse und dadurch ein kontrolliertes Release-Verhalten, also ein kontrolliertes Entfalten der Krone 110 und der Bogeneinrichtung 210 gewährleisten. Die Krone 110 weist eine Länge von 10-15mm, insbesondere 13 mm auf. Die Fixiereinrichtung 105 ermöglicht eine Verbindung zum Herzunterstützungssystem, beispielsweise zu einer Herzpumpe, durch eine Verbindung mit dem Motorgehäuse des Herzunterstützungssystems. Die hier gezeigte Rauten-Form der Entfaltungselemente 120 stellt einen Standard-Schnitt bei Gefäß-Stents dar und ermöglicht einen Laserschnitt aus einem Rohr. Die Füßchen 215 der Bogeneinrichtung 210 weisen je eine axiale Länge von 20-30 mm, insbesondere 24 mm auf. Vorne, also an dem der Krone gegenüberliegenden Ende der Füßchen 215, weist die Bogeneinrichtung 210 einen Rotations-Durchmesservon 20 -30mm, insbesondere 23 mm auf, angepasst an eine menschliche Aortenklappe. Zudem weisen die Füßchen 215 eine atraumatische Form auf, damit beim Einführen oder Entfalten der Füßchen 215 die Aortenwand nicht verletzt wird und die Füßchen 215 automatisch in die Kuspen der Aortenklappe rutschen können. Diese Verankerungsform der Füßchen 215 ist vorteilhaft, da die Erdnuss-Form der Kuspen ein besonders vorteilhaftes Verhältnis aus Anlagefläche und Drehsteifigkeit bietet. Die Einbuchtung der Bogeneinrichtung 210 am Anfang der Füßchen 215, also am mit der Krone 110 verbundenen distalen Ende, ist so ausgelegt, dass bei einem Teil-Release beim Übergang des Einführzustands in den Verankerungszustand, also wenn die Krone 110 noch in gecrimptem Zustand ist, der Ziel-Außendurchmesser der Bogeneinrichtung 210, der dem Rotationsdurchmesser entspricht, bereits erreicht wird.

Die Herstellung des aus der Fixiereinrichtung 105, der Vebindungseinrichtung 115, der Krone 110 und der Bogeneinrichtung 210 bestehenden Rahmens der Vorrichtung 100 kann beispielsweise mittels des hier vorgestellten Herstellverfahrens unter Verwendung eines Formgedächtnismaterials erfolgen. Für den Rahmen wird dazu ein elastisches Material, vorzugsweise Nitinol oder eine andere Formgedächtnislegierung, verwendet. Als Halbzeug für die Bearbeitung bietet sich eine Rohrgeometrie an, welche die gewünschte Wandstärke der späteren Konstruktionselemente, der Krone 110, der Füßchen 215 der Bogeneinrichtung 210, der Verbindungseinrichtung 115 und der Fixiereinrichtung 105 aufweist. Die Konstruktionselemente werden durch ein Verfahren zur Materialwegnahme, vorzugsweise Laserschneiden, realisiert, indem das Rohrvolumen an den nicht benötigten Stellen abgetragen wird. Alternativ sind auch Stanz- und Erodierverfahren oder eine spanende Bearbeitung möglich. Die lasergeschnittene Kontur kann nun im Rahmen einer Wärmebehandlung, beispielsweise mit einer Temperatur über 500°C, in die gewünschte Form, beispielsweise der hier gezeigten Form, gebracht werden. Der Einprägevorgang beschreibt dabei eine plastische Verformung, ohne das Materialversagen auftritt. Die auf diese Weise eingeprägte Form stellt sich nun automatisch ein, sobald die Transformationstemperatur überschritten wird. Diese lässt sich über das Legierungsverhältnis einstellen, und kann im beschriebenen Anwendungsfall unterhalb der Körpertemperatur, vorzugsweise zwischen 0°C und 10°C, liegen. Um Patientengruppen mit unterschiedlichen Größen zu versorgen, kann der so ausgeformte Nitinolrahmen der Vorrichtung 100 in verschiedene Größen zur Verfügung gestellt werden, das Fixieren der Vorrichtung 100 mit dem Herzunterstützungssystem kann dazu beispielsweise erst kurz vor dem Beladen der Implantationsvorrichtung, mit der das mit der Vorrichtung 100 verbundene Herzunterstützungssystem eingeführt werden kann, erfolgen. Dadurch kann die richtige, patientenspezifische Größe der Krone 110 und der Füßchen 215 noch kurz vor der Implantation ausgewählt werden. Bei längeren Einsatzzeiten des Herzunterstützungssystems unterstützt die Nitinolstruktur des Rahmens ein Verwachsen mit der Aorta, indem körpereignes Gewebe das biokompatible Material überzieht.

Fig. 7 zeigt eine schematische Darstellung eines Füßchens 215 einer Bogeneinrichtung gemäß einem Ausführungsbeispiel. Gezeigt ist eine Aufsicht auf das Füßchen 215 der Bogeneinrichtung der Vorrichtung. Das hier gezeigte Füßchen entspricht dem obersten Füßchen 215 in der vorhergehenden Fig. 6, mit der beschriebenen bogenförmigen kurzen Einbuchtung mit einer drauffolgenden halbkreisförmigen Ausbeulung, wobei die Ausbeulung keinen exakten Halbkreis darstellt, sondern eine flacheren Halbkreisbogenabschnitt. Die Bogenform des Füßchens kann der Ausformung eines Abschnitts einer menschlichen Herzklappe entsprechen, beispielsweise einer Kuspe der Aortenklappe. Beim Übergang der Vorrichtung vom Einführzustand in den Verankerungszustand kann sich zunächst das Füßchen 215 entfalten. Das Füßchen 215 kann dann zum Ausrichten des Herzunterstützungssystems im Blutgefäß positioniert werden, beispielsweise hinter der Herzklappe in den Kuspen der Aortenklappe. Sollte die gewünschte Positionierung nicht direkt gelingen, können die Füßchen 215 auch erneut zusammengefaltet werden, auch wiederholt, beispielsweise unter Verwendung der Hülse, indem die Hülse wieder nach vorne, also über die Füßchen 215 geschoben wird, und anschließend zum erneuten Positionieren der Füßchen 215 zurückgeschoben wird, wodurch sich die Füßchen 215 entfalten können.

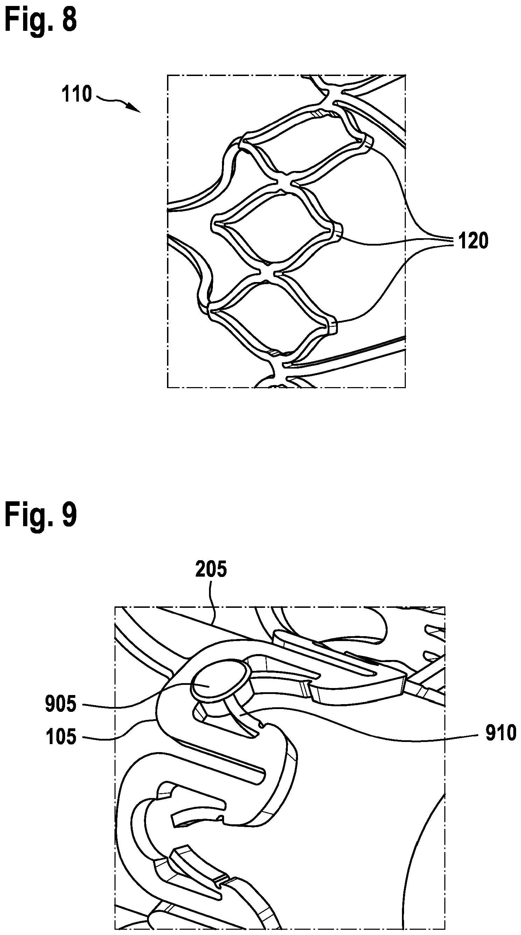

Fig. 8 zeigt eine schematische Darstellung eines Teils einer Krone 110 einer Vorrichtung zum Verankern eines Herzunterstützungssystems in einem Blutgefäß gemäß einem Ausführungsbeispiel. Als Ausschnitt der Krone 110 ist eine Aufsicht auf drei rautenförmig entfaltete Entfaltungselemente 120 der Krone 110 in konischer Form der Krone 110 gezeigt. Die hier gezeigte Schnittkontur der Krone 110 entspricht der nicht optimal kreisrunden Aorten-Anatomie der menschlichen Aorta.

Beim Übergang der Vorrichtung vom Einführzustand in den Verankerungszustand kann beispielsweise zuerst die Bogeneinrichtung und im Anschluss daran die Krone 110 entfaltet werden. Die Form der Krone im entfalteten Zustand wie hier gezeigt kann so ausgeformt sein, dass eine gleichmäßige kraftschlüssige Verbindung zwischen der Krone 110 und der Aorta entsteht. Durch diese Verbindung kann das Herzunterstützungssystem in Position gehalten werden, und eine Dislokation des Herzunterstützungssystems während des Betriebs kann durch die so entstehende Verankerung des Herzunterstützungssystems im Blutgefäß verhindert werden. Dazu ist der Außendurchmesser der Krone 110 im entfalteten Zustand der Krone 110 etwas größer als der Innendurchmesser der Aorta ausgeformt.

Fig. 9 zeigt eine schematische Darstellung einer Fixiereinrichtung 105 einer Vorrichtung zum Verankern eines Herzunterstützungssystems 205 in einem Blutgefäß gemäß einem Ausführungsbeispiel. Die Aufsicht zeigt den mit der Fixiereinrichtung 105 verbundenen Abschnitt des Herzunterstützungssystems 205. Die Fixiereinrichtung 105 ist gemäß einem Ausführungsbeispiel ausgeformt, um die Vorrichtung formschlüssig und/oder kraftschlüssig an das Herzunterstützungssystem 205 zu fixieren. Die Fixierung kann auch stoffschlüssig realisiert werden. Das Fixieren mittels der Fixiereinrichtung 105 kann so ausgebildet sein, dass die Vorrichtung nicht während des Betriebs des Herzunterstützungssystems 205 vom Herzunterstützungssystem 205 gelöst werden kann, die Fixierung kann über die Laufzeit des Herzunterstützungssystems 205 stabil bleiben. Zudem kann die Fixiereinrichtung 105 ausgebildet sein, um während des Betriebs des Herzunterstützungssystems 205 oder während der Operation auftretenden Kräfte aufzunehmen. Die Fixiereinrichtung 105 kann ausgeformt werden, um das Positionieren des Herzunterstützungssystems im Blutgefäß zu unterstützen. Die Position zwischen dem Herzunterstützungssystem 205 und der Fixiereinrichtung kann über eine rotatorische Bewegung in beide Richtung justierbar und über 360° schrittweise variabel gestaltet sein, die Fixiereinrichtung 105 und das Herzunterstützungssystem 205 können entsprechend vor dem formschlüssigen Fixieren gegenüber einander beweglich sein.

Zum formschlüssigen und/oder kraftschlüssigen Fixieren des Herzunterstützungssystems 205 gemäß einem Ausführungsbeispiel können die Fixiereinrichtung 105 und das Herzunterstützungssystem 205 somit Merkmale aufweisen, die ein Einrasten des Herzunterstützungssystems in der Vorrichtung und damit ein Verankern des Herzunterstützungssystems 205 an der Vorrichtung ermöglichen. Dazu können das Herzunterstützungssystem 205 und/oder die Fixiereinrichtung 105 zumindest ein aufgetragenes Element 905 zum formschlüssigen Verbinden der Vorrichtung und des Herzunterstützungssystems 205 mittels der Fixiereinrichtung 105 aufweisen. In der vorliegenden Fig. 9 ist das Element beispielhaft auf dem Herzunterstützungssystem aufgetragen, rund ausgeführt und weist im Querschnitt einen Hinterschnitt auf, zum Eingreifen der entsprechend des Elements 905 ausgeformten und hier gezeigten Klippverbindung der Fixiereinrichtung 105. Die Klippverbindung weist hier zwei Sicherungsfedern 910 auf, um die Fixiereinrichtung einfach mit dem Herzunterstützungssystem verbinden zu können, und nach dem Eingreifen des formschlüssigen Elements eine zusätzliche Fixierung durch dir Sicherungsfedern 910 zu erreichen. Unabhängig vom rotatorischen Versatz des aufgetragenen Elements 905 gegenüber der entsprechenden Aussparung kann ein Verrasten der Fixiereinrichtung mit dem Herzunterstützungssystem 205 realisiert werden, das hier in Form der Sicherungsfedern 910 ausgeführt ist.

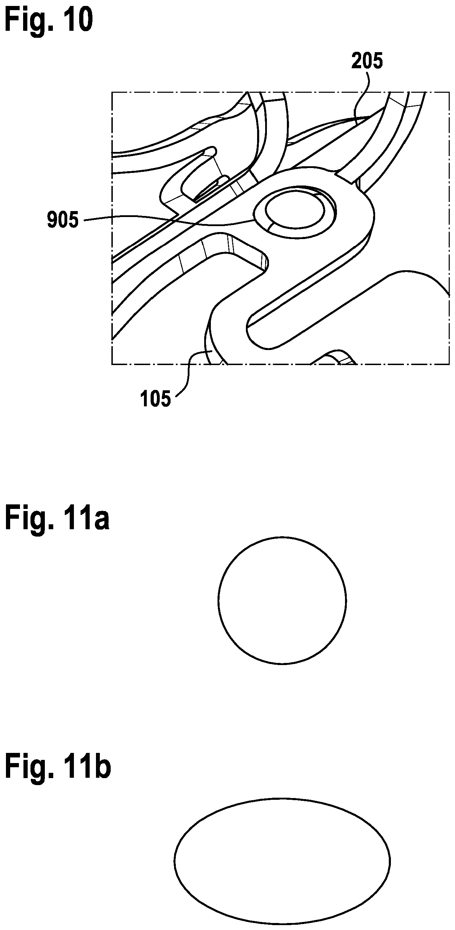

In den folgenden Figuren 10 bis 17 sind je verschiedene Ausführungsbeispiele zum formschlüssigen und/oder kraftschlüssigen Fixieren des Herzunterstützungssystems 205 mit der Vorrichtung gezeigt.

Fig. 10 zeigt eine schematische Darstellung einer Fixiereinrichtung 105 einer Vorrichtung zum Verankern eines Herzunterstützungssystems 205 in einem Blutgefäß gemäß einem Ausführungsbeispiel. In der Aufsicht ist ein mit einem Abschnitt der Fixiereinrichtung 105 verbundene Abschnitt des Herzunterstützungssystems 205 gezeigt. Auf dem Herzunterstützungssystem ist ein rundes Element 905 aufgetragen, und ein Abschnitt der Fixierausrichtung weist eine dem Element 905 entsprechende Aussparung auf, zum forschlüssigen Eingreifen der Fixiereinrichtung 105, um das Herzunterstützungssystem 205 sicher an die Vorrichtung zu fixieren.

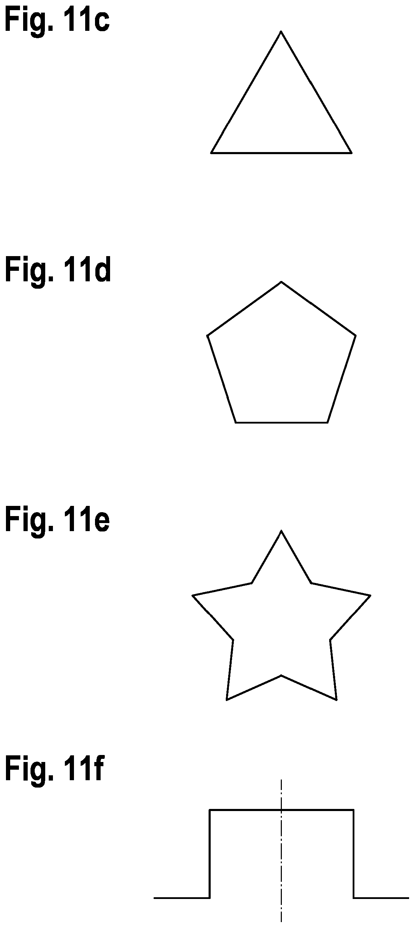

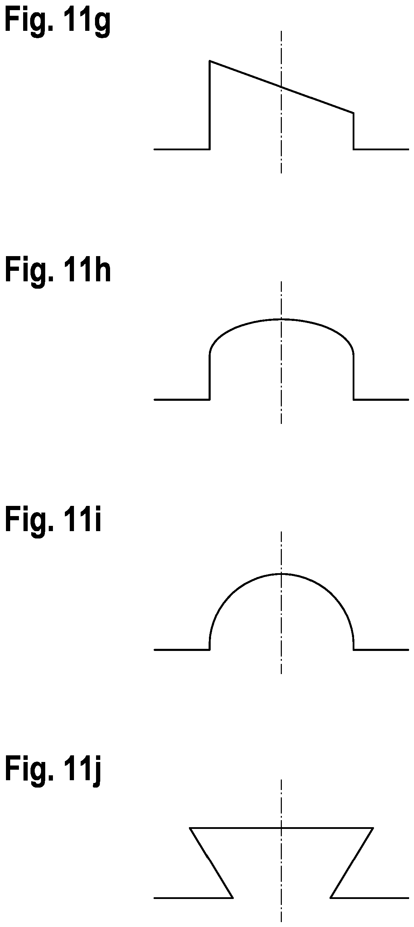

Fig. 11a bis 11j zeigen je eine schematische Darstellung einer Ausformung einer formschlüssigen Fixierung einer Vorrichtung zum Verankern eines Herzunterstützungssystems in einem Blutgefäß gemäß einem Ausführungsbeispiel. Gezeigt ist jeweils eine mögliche Ausformung des in den beiden vorhergehenden Figuren 9 und 10 gezeigten aufgetragenen Elements zum formschlüssigen Verbinden des Herzunterstützungssystems mit der Vorrichtung mittels des Ineinandergreifens des aufgetragenen Elements und einer entsprechenden Aussparung oder Ausformung als Gegenpart. In der Art einer Positiv-und Negativausführung kann ein solches auftragendes Elemente zum formschlüssigen Fixieren sowohl auf dem Herzunterstützungsystem, beispielsweise dem Gehäuse der Pumpe, als auch auf dem Anbindungsmechanismus der Vorrichtung, also auf der Fixiereinrichtung realisiert werden. Die Figuren 11a bis 11e zeigen die verschiedenen Formen, die das Element zum formschlüssigen Fixieren durch die Ausformung annehmen kann in einer schematischen Draufsicht, und die Figuren 11f bis 11j zeigen verschiedene Querschnitte, die ein solches Element aufweisen kann. Das Element kann gemäß der in Fig. 11a gezeigten Form als Kreis ausgeformt sein, oder gemäß der in Fig. 11b gezeigten Form eine ovale Form aufweisen, gemäß Fig. 11c kann das Element ein Dreieck sein, oder ein Vieleck wie in Fig. 11d gezeigt. Zudem kann das Element wie in Fig. 11 e gezeigt als Stern ausgeformt sein. Das Element kann einen Querschnitt in der Form eines Rechtecks wie in Fig. 11f gezeigt aufweisen, oder die in Fig. 11g gezeigte Abflachung des Querschnitts, die in Fig. 11 h gezeigte Abrundung des Querschnitts, den in Fig. 11i gezeigten Halbkreis des Querschnitts, oder den in Fig. 11j gezeigten Hinterschnitt des Querschnitts aufweisen. Auch Kombinationen der gezeigten Ausformungen können zum Ausformen des Elements verwendet werden.

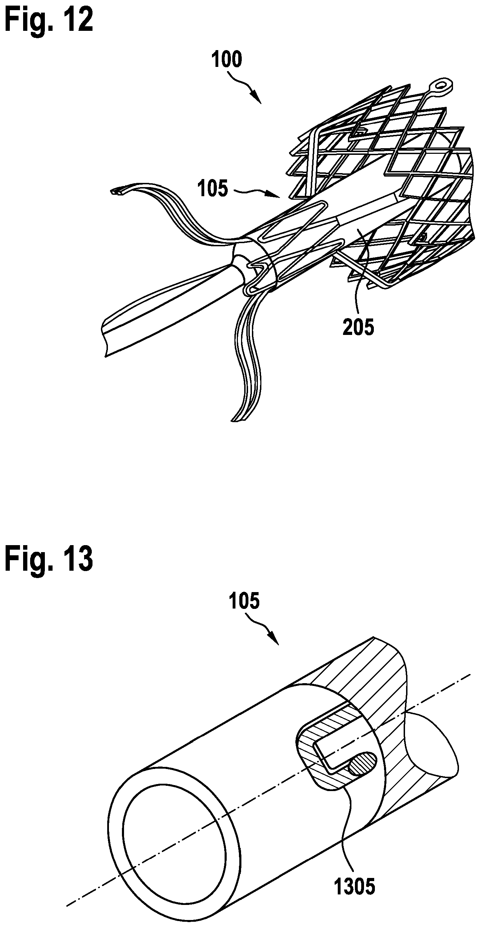

Fig. 12 zeigt eine schematische Darstellung einer kraftschlüssigen Fixierung einer Vorrichtung 100 zum Verankern eines Herzunterstützungssystems 205 in einem Blutgefäß an ein Herzunterstützungssystem 205 gemäß einem Ausführungsbeispiel. Die Aufsicht zeigt einen Abschnitt des Herzunterstützungssystems 205 und die fixierte Vorrichtung 100. Die Vorrichtung 100 weist dazu eine Fixierungseinrichtung mit mehreren rautenförmigen Fixierungselementen auf, die ausgeformt sind, um ein gegeneinander Verschieben des Herzunterstützungssystems 205 und der Vorrichtung 100 zu verhindern. Die Fixierungselemente liegen dazu gemäß dem hier gezeigten Ausführungsbeispiel auf einem Abschnitt des Herzunterstützungssystems 205 auf und klemmen die Vorrichtung gegen das Herzunterstützungssystems 205, um die Vorrichtung kraftschlüssig an das Herzunterstützungssystem 205 zu fixieren.

Fig. 13 zeigt eine schematische Darstellung eines Teils einer Fixiereinrichtung 105 einer Vorrichtung zum Verankern eines Herzunterstützungssystems in einem Blutgefäß gemäß einem Ausführungsbeispiel. Es ist eine Aufsicht auf eine formschlüssig realisierte Verbindung der Vorrichtung mit dem Herzunterstützungssystem gezeigt, die in Form einer Bajonettverbindung realisiert ist. Die Fixiereinrichtung 105 umfasst dazu hier einen Bajonettanschluss in der Form eines Hakens.

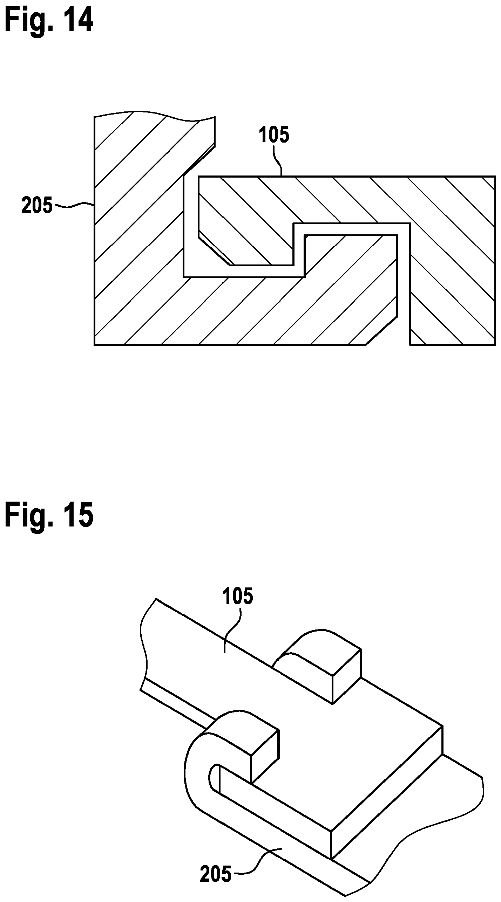

Fig. 14 zeigt eine schematische Darstellung eines Teils einer Fixiereinrichtung einer Vorrichtung zum Verankern eines Herzunterstützungssystems 205 in einem Blutgefäß gemäß einem Ausführungsbeispiel als Querschnitt. Die Fixiereinrichtung 105 ist hier zum formschlüssigen Fixieren ausgeformt, das Herzunterstützungssystem weist eine entsprechende Ausformung auf. Die Fixiereinrichtung und das Herzunterstützungssystem weisen dazu jeweils eine Nut zum Fixieren auf.

Fig. 15 zeigt eine schematische Darstellung eines Teils einer Fixiereinrichtung 105 einer Vorrichtung zum Verankern eines Herzunterstützungssystems 205 in einem Blutgefäß gemäß einem Ausführungsbeispiel. Es ist beispielhaft je ein Abschnitt der Fixiereinrichtung 105 und des Herzunterstützungssystems 205 gezeigt, wobei die Abschnitte formschlüssig ineinander aufgreifen. Die Aufsicht zeigt, die formschlüssige Verbindung der Fixiereinrichtung 105 mit dem Herzunterstützungssystem durch ein Eingreifen der Fixiereinrichtung 105 in zwei Haken des Herzunterstützungssystems 205.

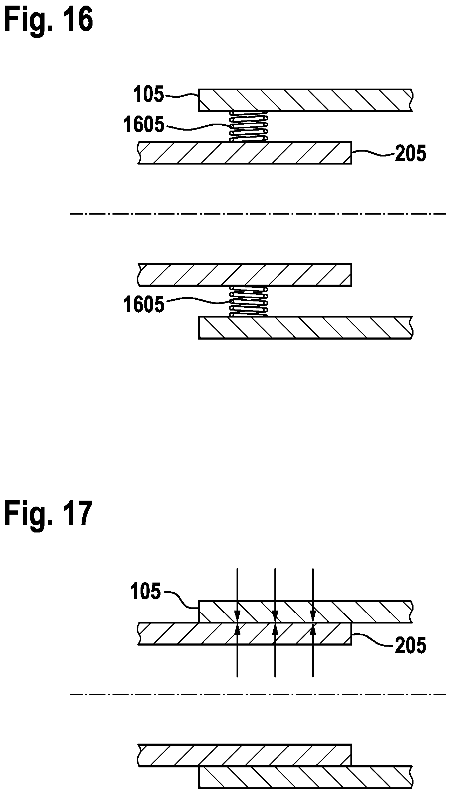

Fig. 16 zeigt eine schematische Darstellung eines Teils einer Fixiereinrichtung einer Vorrichtung zum Verankern eines Herzunterstützungssystems 205 in einem Blutgefäß gemäß einem Ausführungsbeispiel. Gezeigt ist ein Querschnitt einer kraftschlüssigen Verbindung des Herzunterstützungssystems 205 mit der Fixiereinrichtung 105, die hier durch eine Federverbindung mittels zweier Federn 1605 zwischen dem Herzunterstützungssystem 205 und der Fixiereinrichtung 105 als Federklemme realisiert wird.

Fig. 17 zeigt eine schematische Darstellung eines Teils einer Fixiereinrichtung einer Vorrichtung zum Verankern eines Herzunterstützungssystems 205 in einem Blutgefäß gemäß einem Ausführungsbeispiel. Es ist der Querschnitt einer Pressverbindung als kraftschlüssige Verbindung des Herzunterstützungssystems 205 mit der Fixiereinrichtung 105 gezeigt. Die hier gezeigte Montage der Pressverbindung kann mittels Einpresskraft erzeugt werden, oder mittels eines Temperaturunterschieds zwischen dem Herzunterstützungssystem 205 und der Fixiereinrichtung 105, also durch ein Pressfügen mittels eines Temperaturunterschiedes zwischen der Fixiereinrichtung 105 und dem Herzunterstützungssystem 205 und der sich bei dem anschließenden Temperaturausgleich einstellenden Pressung.

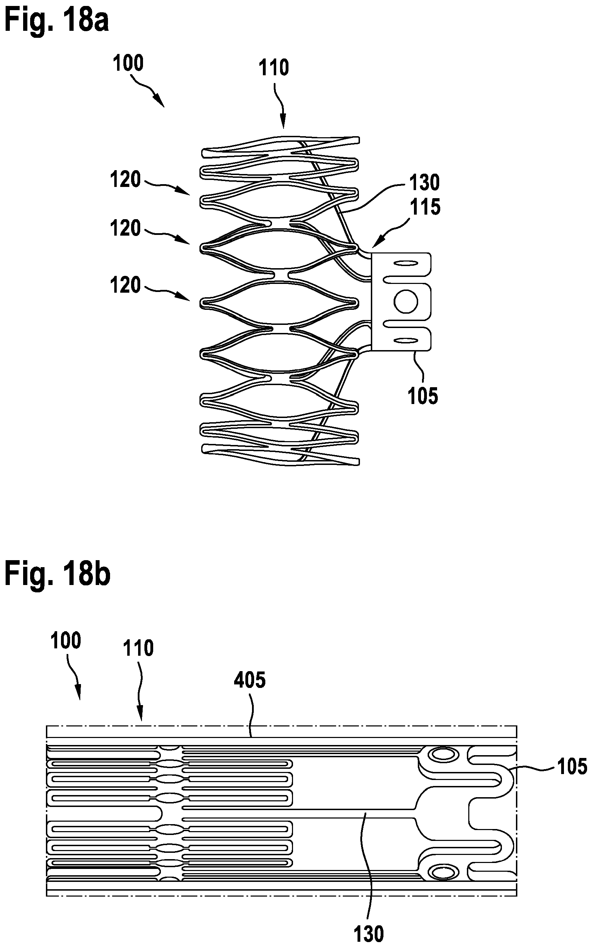

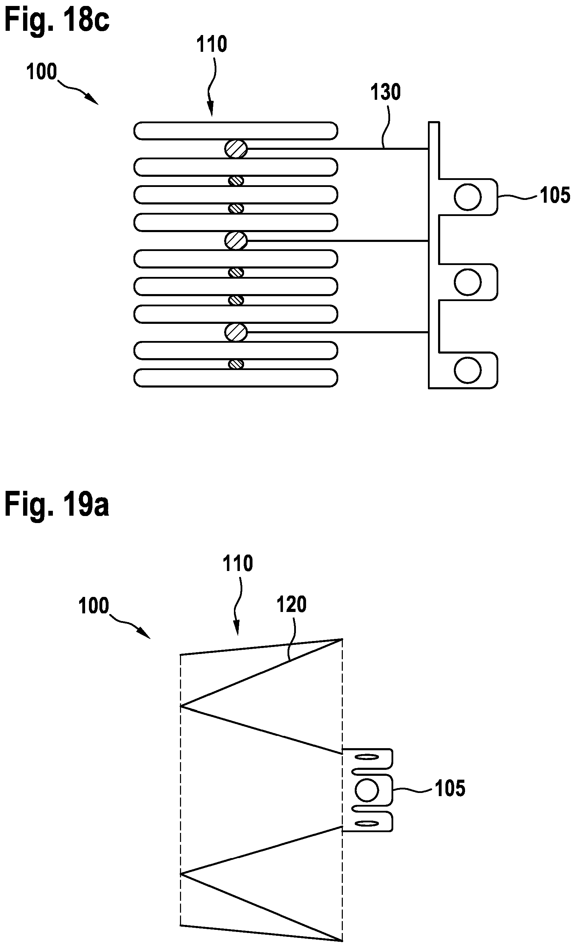

Fig. 18a bis 18c zeigen je eine schematische Darstellung einer Vorrichtung 100 zum Verankern eines Herzunterstützungssystems in einem Blutgefäß gemäß einem Ausführungsbeispiel, wobei je unterschiedliche Situationen der Vorrichtung 100 gezeigt sind. Fig. 18a zeigt eine Aufsicht auf die entfaltete Vorrichtung 100, wie es bereits anhand von Fig. 1 beschrieben ist. Fig. 18b zeigt einen Ausschnitt der in Fig. 18a gezeigten Vorrichtung in der Hülse 405 im Einführzustand, wie es bereits anhand von Fig. 4 beschrieben ist. Fig. 18c zeigt eine Seitenansicht der Vorrichtung 100. Die hier gezeigten Situationen stellen beispielhaft den im Einführzustand erforderlichen Platzbedarf der Vorrichtung 100 je nach Ausformung der Vorrichtung 100 dar. Die nachfolgenden Figuren 19a bis 19c zeigen die hier beschriebenen Situationen entsprechend einer alternativen Ausformung der Krone 110 der Vorrichtung 100 gemäß einem Ausführungsbeispiel.

Fig. 18a zeigt eine Seitenansicht der Vorrichtung 100 gemäß einem Ausführungsbeispiel. Die Vorrichtung 100 umfasst die Fixiereinrichtung 105, die Verbindungseinrichtung 115 und die Krone 110, wobei die Krone 110 aus einer Mehrzahl an miteinander gekoppelten Entfaltungselementen 120 ausgeformt ist. Jedes der Entfaltungselemente 120 weist zwei an ihren Enden verbundene Entfaltungsstäbe auf, die im hier gezeigten Entfaltungszustand rautenförmig sind. Die Verbindungseinrichtung 115 besteht aus vier Biegestreben 130, wobei ein erstes Ende jeder Biegestrebe 130 an der Fixiereinrichtung 105 befestigt ist und wobei ein zweites Ende jeder Biegestrebe 130 an einer Verbindung zwischen zwei benachbarten Entfaltungselementen 120 befestigt ist.

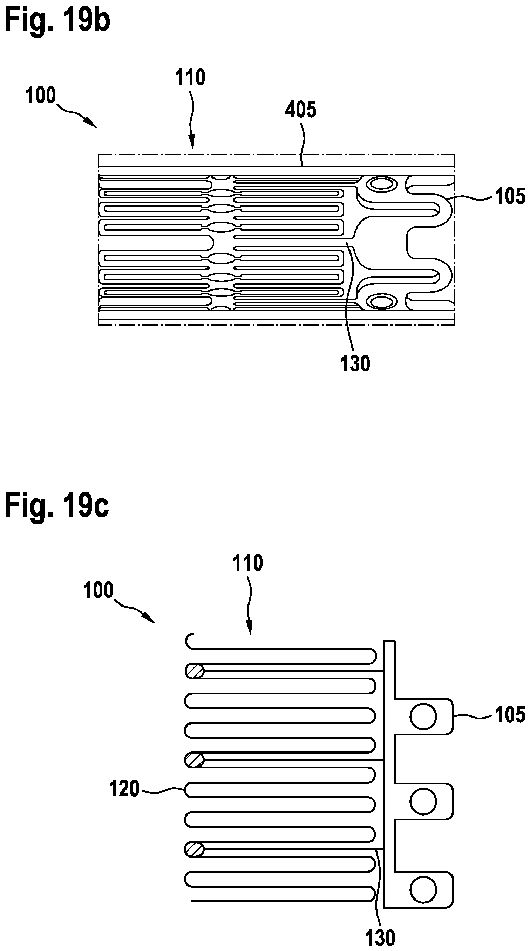

Fig. 18b zeigt die in Fig. 18a beschrieben Vorrichtung 100 im Einführzustand. Die Krone 110 ist hier zum Einführen zusammengefaltet und in die Hülse 405 eingeführt. Durch die beschriebene Ausformung der Krone 110 und die Befestigungsart der Biegestreben 130 weist die Vorrichtung 100, auch Anker genannt, einen vergleichsweise hohen axialen Platzbedarf auf, beispielsweise im Vergleich zu dem in Fig. 19b gezeigten Ausführungsbeispiel der Vorrichtung 100. Die Biegestreben 130 sind gemäß diesem Ausführungsbeispiel nicht vollständig unterhalb oder zwischen den Entfaltungselementen der Krone 110 eingefaltet, sondern benötigen im Einführzustand einen bestimmten axialen Platz, der in dieser Figur durch den Abstand zwischen der geladenen Krone 110 und der Fixiereinrichtung 105 gezeigt ist.

Fig. 18c zeigt schematisch eine Darstellung der Vorrichtung 100 im abgewickelten Zustand. Die Vorrichtung 100 weist entsprechend nicht die zylinderförmige Ausformung des Einführzustandes oder die entfaltete Ausformung des Verankerungszustands auf, der hier gezeigte abgewickelte Zustand zeigt beispielhaft den Platzbedarf durch die Verbindung und Ausformung der Krone 110 und der Biegestreben 130 der Verbindungseinrichtung.

Fig. 19a bis 19c zeigen je eine schematische Darstellung einer Vorrichtung 100 zum Verankern eines Herzunterstützungssystems in einem Blutgefäß gemäß einem Ausführungsbeispiel. Die in den Fig. 19a bis 19c gezeigten Situationen zeigen je die Vorrichtung 100, wie sie anhand von Fig. 19a beschrieben wird. Gezeigt ist eine Ausformung der Krone 110 und der Verbindungseinrichtung 115, mit der die Vorrichtung 100 im geladenen Zustand, als im dem Einführzustand entsprechenden zusammengefalteten Zustand einen vergleichsweise geringen axialen Platzbedarf aufweist, was beispielsweise vorteilhaft sein kann, wenn die Vorrichtung in Verbindung mit einem Herzunterstützungssystem verwendet wird, für das in diesem Bereich zusätzlicher Bauraum vorteilhaft ist, beispielsweise zum Betreiben eines Motors einer Herzpumpe. Die Einsparung im axialen Platz kann durch eine modifizierte Geometrie der Krone 110 und der Verbindungseinrichtung 115 erreicht werden. Durch die Ausformung der Krone 110, die Befestigung der Biegestreben 130, sowie durch lange Biegestreben 130 und deren Richtungsänderung kann weitgehend vermieden werden, dass der Anker, also die Vorrichtung 100, beim Laden unnötig axialen Bauraum beansprucht. Zudem entfaltet sich die Krone 110 gemäß dem hier gezeigten Ausführungsbeispiel an der Stelle, an der sie sich auch im geladenen Zustand befindet.

Fig. 19a zeigt die Vorrichtung 100 in der Seitenansicht im entfalteten Zustand, wobei die Vorrichtung 100 gemäß diesem Ausführungsbeispiel einen geringen axialen Platzbedarf aufweist. Die Krone 110 ist aus dem zumindest einen Entfaltungselement mit einer Mehrzahl an mäanderartig angeordneten Schlaufen ausgeformt, wie in Fig. 19c gezeigt. Die Krone 110 ist an der Fixiereinrichtung 105 befestigt.

Fig. 19b zeigt die in Fig. 19a beschrieben Vorrichtung 100 im Einführzustand. Die Krone 110 ist hier zum Einführen gecrimpt, und in die Hülse 405 eingeführt. Durch die Ausformung der Krone 110 und die Befestigungsart der Biegestreben 130 weist die Vorrichtung 100, auch Anker genannt, einen vergleichsweise geringen axialen Platzbedarf auf, beispielsweise im Vergleich zu dem in Fig. 18b gezeigten Ausführungsbeispiel der Vorrichtung 100. Die Biegestreben 130 sind gemäß diesem Ausführungsbeispiel zwischen den Entfaltungselementen der Krone 110 eingefaltet, sie benötigen im Einführzustand nur einen sehr geringen axialen Platz, der in dieser Figur durch den geringen Abstand zwischen der gecrimpten Krone 110 und der Fixiereinrichtung 105 gezeigt ist.

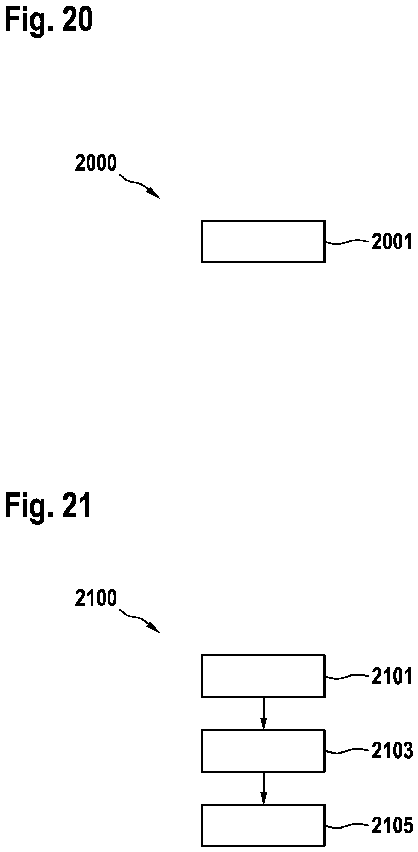

Fig. 19c zeigt schematisch eine Darstellung der Vorrichtung 100 im abgewickelten Zustand als weitere Situation. Die beschriebene Ausformungen des Entfaltungselements 120 mit einer Mehrzahl mäanderartiger Schlaufen und die beschriebene Verbindung der Biegestreben 130 mit der Krone 110 sind in dieser Figur deutlich gezeigt. Somit kann die Krone 110 aus nur einem einzigen Entfaltungselement 120 ausgeformt sein. Die Biegestreben 130 der Verbindungseinrichtung sind innerhalb der mäanderartigen Schlaufen angeordnet, wobei ein erstes Ende jeder Biegestrebe 130 an der Fixiereinrichtung 105 befestigt ist und wobei ein zweites Ende der Biegestrebe 130 an einem der Fixiereinrichtung 105 abgewandten Ende der Krone 110 befestigt ist. Diese Verbindung der Biegestreben 130 mit der Krone 110 ist besonders im Hinblick auf den axialen Platzbedarf der Vorrichtung 100 im Einführzustand vorteilhaft, wie in der vorhergehenden Situation der Vorrichtung 100 in Fig. 19b gezeigt. Fig. 20 zeigt ein Ablaufdiagramm eines Verfahrens 2000 zum Betreiben einer Vorrichtung zum Verankern eines Herzunterstützungssystems in einem Blutgefäß (nicht beansprucht). Das Verfahren 2000 weist zumindest einen Schritt 2001 des Entfaltens des Entfaltungselements der Krone der Vorrichtung bei dem Übergang von dem Einführzustand in den Verankerungszustand zum Vergrößern des Durchmessers der Krone auf.

Fig. 21 zeigt ein Ablaufdiagramm eines Herstellverfahrens 2100 zum Herstellen einer Vorrichtung zum Verankern eines Herzunterstützungssystems in einem Blutgefäß gemäß einem Ausführungsbeispiel. Die Vorrichtung kann einen Einführzustand zum Einführen des Herzunterstützungssystems in das Blutgefäß einnehmen, und die Vorrichtung kann einen Verankerungszustand einnehmen, um das Herzunterstützungssystems in dem Blutgefäß zu verankern. Das Herstellverfahren 2100 der Vorrichtung weist zumindest einen Schritt 2101 des Bereitstellens, einen Schritt 2103 des Ausformens und einen Schritt 2105 des Wärmebehandelns auf. Im Schritt 2101 des Bereitstellens wird ein Halbzeug aus einem Formgedächtnismaterial bereitgestellt. Im Schritt 2103 des Ausformens wird eine Fixiereinrichtung zum Fixieren der Vorrichtung an das Herzunterstützungssystem ausgeformt. Zudem wird eine Krone aus zumindest einem Entfaltungselement ausgeformt, wobei das Entfaltungselement dazu ausgebildet ist, um sich bei einem Übergang von dem Einführzustand in den Verankerungszustand zu entfalten zum Vergrößern des Durchmessers der Krone, um die Vorrichtung in dem Blutgefäß zu verankern. Ferner wird eine Verbindungseinrichtung dazu ausgeformt, die Krone mit der Anbindungseinrichtung zu verbinden. Die Fixierungseinrichtung, die Krone und die Verbindungseinrichtung werden aus dem im Schritt 2101 bereitgestellten Halbzeug ausgeformt. Im Schritt 2105 des Wärmebehandelns werden die Fixierungseinrichtung, die Krone und die Verbindungseinrichtung wärmebehandelt, um die Form des Verankerungszustands einzuprägen.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

Zusammenfassend sind insbesondere die folgenden bevorzugten Merkmale der Erfindung festzuhalten: Eine Vorrichtung 100 zum Verankern eines Herzunterstützungssystems in einem Blutgefäß kann einen Einführzustand zum Einführen des Herzunterstützungssystems in das Blutgefäß und einen Verankerungszustand einnehmen, um das Herzunterstützungssystems in dem Blutgefäß zu verankern. Die Vorrichtung 100 weist zumindest eine Fixiereinrichtung 105 zum Fixieren der Vorrichtung 100 an das Herzunterstützungssystem 205, eine Krone 110 und eine Verbindungseinrichtung 115 auf. Die Krone 110 ist aus zumindest einem Entfaltungselement 120 ausgeformt. Das Entfaltungselement 120 ist dazu ausgebildet ist, um sich bei einem Übergang von dem Einführzustand in den Verankerungszustand zu entfalten zum Vergrößern des Durchmessers der Krone 110, um die Vorrichtung 100 in dem Blutgefäß zu verankern. Die Verbindungseinrichtung 115 ist dazu ausgeformt, die Krone 110 mit der Fixiereinrichtung 105 zu verbinden.

## Patentansprüche

1. Vorrichtung (100) zum Verankern eines Herzunterstützungssystems (205) in einem Blutgefäß, die einen Einführzustand zum Einführen des Herzunterstützungssystems (205) in das Blutgefäß und einen Verankerungszustand zum Verankern des Herzunterstützungssystems (205) in dem Blutgefäß einnehmen kann und die eine Fixiereinrichtung (105) zum Fixieren an das Herzunterstützungssystem (205) sowie eine ringförmige Krone (110) aufweist, deren Durchmesser bei einem Übergang der Vorrichtung (100) von dem Einführzustand in den Verankerungszustand vergrößert wird, um das Herzunterstützungssystem (205) in dem Blutgefäß zu verankern, wobei die Vorrichtung (100) zum Verankern des Herzunterstützungssystems (205) eine Verbindungseinrichtung (115) hat, die dazu ausgeformt ist, die Krone (110) mit der Fixiereinrichtung (105) zu verbinden und die mehrere Biegestreben (130) enthält, die dazu ausgebildet sind, durch Bewegen einer in dem Katheter relativ zu der Krone (110) beweglich verlagerbar angeordneten Hülse (405) bei einem Übergang von dem Einführzustand in den Verankerungszustand aufzuklappen, um ein Entfalten der Krone (110) zu ermöglichen, wobei die Biegestreben (130) jeweils ein erstes Ende haben, das an der Fixiereinrichtung befestigt ist,
**dadurch gekennzeichnet, dass**
die ringförmige Krone (110) mehrere Entfaltungselemente (120) aufweist, die für das Entfalten bei einem Übergang der Vorrichtung (100) von dem Einführzustand in den Verankerungszustand zum Vergrößern des Durchmessers der Krone (110) ausgebildet sind,
wobei ein jedes Entfaltungselement (120) zwei an ihren Enden verbundene Entfaltungsstäbe (125) aufweist,
wobei die Entfaltungsstäbe (125) eines jeden Entfaltungselements (120) in einem auf einer Kreisbahn liegenden mittigen Abschnitt jeweils mit einem Entfaltungsstab (125) eines zu dem Entfaltungselement (120) benachbart angeordneten Entfaltungselement (120) in einem auf einer Kreisbahn liegenden mittigen Abschnitt verbunden sind,
wobei jeweils zwei zueinander benachbart angeordnete Entfaltungsstäbe (125) im Einführzustand der Vorrichtung (100) eine geringere Entfernung zueinander aufweisen als im Verankerungszustand der Vorrichtung (100), wobei die Biegestreben (130) jeweils ein zweites Ende aufweisen, das an eine Verbindungsstelle zweier zueinander benachbart angeordneter Entfaltungsstäbe (125) oder an ein der Fixiereinrichtung (105) abgewandtes Ende zweier an diesem Ende miteinander verbundener Entfaltungsstäbe (125) der Krone (110) angeschlossen ist, und wobei
die in dem Katheter relativ zu der Krone (110) beweglich verlagerbar angeordnete Hülse (405) dazu ausgeformt ist, in dem Einführzustand der Vorrichtung (100) zum Verankern des Herzunterstützungssystems (205) die Krone (110) zu umschließen und diese zum Einleiten des Übergangs in den Verankerungszustand freizugeben.

2. Vorrichtung gemäß Anspruch 1, **dadurch gekennzeichnet, dass** die Entfaltungselemente (120) aus einem Formgedächtnismaterial ausgeformt sind.

3. Vorrichtung (100) zum Verankern eines Herzunterstützungssystems (205) in einem Blutgefäß, die einen Einführzustand zum Einführen des Herzunterstützungssystems (205) in das Blutgefäß und einen Verankerungszustand zum Verankern des Herzunterstützungssystems (205) in dem Blutgefäß einnehmen kann und die eine Fixiereinrichtung (105) zum Fixieren an das Herzunterstützungssystem (205) sowie eine ringförmige Krone (110) aufweist, deren Durchmesser bei einem Übergang der Vorrichtung (100) von dem Einführzustand in den Verankerungszustand vergrößert wird, um das Herzunterstützungssystem (205) in dem Blutgefäß zu verankern, wobei die Vorrichtung (100) zum Verankern des Herzunterstützungssystems (205) eine Verbindungseinrichtung (115) hat, die dazu ausgeformt ist, die Krone (110) mit der Fixiereinrichtung (105) zu verbinden und die mehrere Biegestreben (130) enthält, die dazu ausgebildet sind, durch Bewegen einer in dem Katheter relativ zu der Krone (110) beweglich verlagerbar angeordneten Hülse (405) bei einem Übergang von dem Einführzustand in den Verankerungszustand aufzuklappen, um ein Entfalten der Krone (110) zu ermöglichen, wobei die Biegestreben (130) jeweils ein erstes Ende haben, das an der Fixiereinrichtung befestigt ist,
**dadurch gekennzeichnet, dass**
die ringförmige Krone (110) aus einem Entfaltungselement (120) besteht, das mehrere mäanderförmige Schlaufen aufweist, die für das Entfalten bei einem Übergang der Vorrichtung (100) von dem Einführzustand in den Verankerungszustand zum Vergrößern des Durchmessers der Krone (110) ausgebildet sind,
wobei die Schlaufen im Einführzustand eine geringere Entfernung zueinander aufweisen als im Verankerungszustand,
wobei die Verbindungseinrichtung (115) mehrere innerhalb der mäanderförmigen Schlaufen angeordnete Biegestreben (130) enthält, die jeweils ein erstes Ende haben, das an der Fixiereinrichtung befestigt ist, und ein zweites Ende aufweisen, das an ein der Fixiereinrichtung (105) abgewandtes Ende der Krone (110) angeschlossen ist, und wobei
die in dem Katheter relativ zu der Krone (110) beweglich verlagerbar angeordnete Hülse (405) dazu ausgeformt ist, in dem Einführzustand der Vorrichtung (100) zum Verankern des Herzunterstützungssystems (205) die Krone (110) zu umschließen und diese zum Einleiten des Übergangs in den Verankerungszustand freizugeben.

4. Vorrichtung gemäß Anspruch 3, **dadurch gekennzeichnet, dass** das Entfaltungselement (120) aus einem Formgedächtnismaterial ausgeformt ist.

5. Vorrichtung gemäß einem der vorangegangenen Ansprüche, **gekennzeichnet durch** eine Bogeneinrichtung (210) mit zumindest einem Füßchen (215), wobei die Bogeneinrichtung (210) dazu ausgebildet ist, sich bei dem Übergang von dem Einführzustand in den Verankerungszustand zum Positionieren des zumindest einen Füßchens (215) im Blutgefäß zu entfalten, und wobei die Bogeneinrichtung (210) mit der Krone (110) verbunden ist oder wobei die Bogeneinrichtung (210) eine Bogenfixierungseinrichtung (305) zum Fixieren der Bogeneinrichtung (210) an das Herzunterstützungssystem (205) aufweist.

6. Vorrichtung gemäß Anspruch 5, **dadurch gekennzeichnet, dass** die Bogeneinrichtung (210) drei Füßchen (215) aufweist, insbesondere wobei die Füßchen (215) zum Positionieren der Füßchen (215) in je einer Kuspe einer Herzklappe ausgeformt sind.

7. Vorrichtung gemäß einem der vorangegangenen Ansprüche, **dadurch gekennzeichnet, dass** die Vorrichtung (100) im Einführzustand zylinderförmig ist.

8. Vorrichtung gemäß einem der vorangegangenen Ansprüche, **dadurch gekennzeichnet, dass** die Krone (110) und die Fixiereinrichtung (105) entlang einer Längsachse erstreckt sind, die der Achse eines Katheters entsprechen kann, und das Entfaltungselement (120) im Verankerungszustand eine Schrägstellung gegenüber dieser Längsachse der Vorrichtung (100) aufweist.

9. Vorrichtung gemäß einem der vorangegangenen Ansprüche, **dadurch gekennzeichnet, dass** die Fixiereinrichtung (105) ausgeformt ist, um die Vorrichtung (100) formschlüssig und/oder kraftschlüssig an das Herzunterstützungssystem (205) zu fixieren.

10. Anordnung mit einem Herzunterstützungssystem (205), das eine in einen Katheter einführbare Herzpumpe mit einem Pumpenrad und mit einem Ansaugschlauch aufweist, und mit einer nach einem der Ansprüche 1 bis 9 ausgebildeten Vorrichtung (100).

11. Herstellverfahren (2100) zum Herstellen einer nach einem der Ansprüche 1 bis 10 ausgebildeten Vorrichtung (100), wobei das Herstellverfahren zumindest folgende Schritte aufweist:
Bereitstellen (2101) eines Halbzeugs aus einem Formgedächtnismaterial;
Ausformen (2103) der Fixiereinrichtung (105) und Ausformen der Krone (110) sowie Ausformen der Verbindungseinrichtung (115) aus dem Halbzeug; und
Wärmebehandeln (2105) der Fixierungseinrichtung, der Krone (110) und der Verbindungseinrichtung (115), um die Form des Verankerungszustands einzuprägen.

## Claims

1. Device (100) for anchoring a ventricular assist system (205) in a blood vessel, which device can assume an insertion state for inserting the ventricular assist system (205) into the blood vessel, and an anchoring state for anchoring the ventricular assist system (205) in the blood vessel, and which comprises a securing means (105) for securing to the ventricular assist system (205), as well as an annular crown (110), the diameter of which is increased during a transition of the device (100) from the insertion state to the anchoring state in order to anchor the ventricular assist system (205) in the blood vessel, the device (100) for anchoring the ventricular assist system (205) having a connection means (115) which is designed to connect the crown (110) to the securing means (105), and which contains a plurality of bending struts (130) which are designed to fold out, during a transition from the insertion state to the anchoring state, by movement of a sleeve (405) arranged so as to be movable in the catheter relative to the crown (110), in order to allow the crown (110) to be unfurled, the bending struts (130) each having a first end which is fastened to the securing means,
**characterized in that**
the annular crown (110) comprises a plurality of unfurling elements (120) which are designed for unfurling during a transition of the device (100) from the insertion state to the anchoring state in order to increase the diameter of the crown (110),
each unfurling element (120) comprising two unfurling rods (125) connected at the ends thereof,
the unfurling rods (125) of each unfurling element (120), in a central portion lying on a circular path, each being connected to an unfurling rod (125) of an unfurling element (120) arranged adjacent to the unfurling element (120) in a central portion lying on a circular path,
in each case two adjacent unfurling rods (125) having a smaller distance from one another in the insertion state of the device (100) than in the anchoring state of the device (100), the bending struts (130) each having a second end which is connected to a connection point of two adjacent unfurling rods (125) or to an end, remote from the securing means (105), of two unfurling rods (125) of the crown (110) which are interconnected at this end, and
the sleeve (405) arranged so as to be movable in the catheter relative to the crown (110) being designed to enclose the crown (110) in the insertion state of the device (100) for anchoring the ventricular assist system (205) and to release it to initiate the transition into the anchoring state.

2. Device according to claim 1, **characterized in that** the unfurling elements (120) are formed from a shape memory material.

3. Device (100) for anchoring a ventricular assist system (205) in a blood vessel, which device can assume an insertion state for inserting the ventricular assist system (205) into the blood vessel, and an anchoring state for anchoring the ventricular assist system (205) in the blood vessel, and which comprises a securing means (105) for securing to the ventricular assist system (205), as well as an annular crown (110), the diameter of which is increased during a transition of the device (100) from the insertion state to the anchoring state in order to anchor the ventricular assist system (205) in the blood vessel, the device (100) for anchoring the ventricular assist system (205) having a connection means (115) which is designed to connect the crown (110) to the securing means (105), and which contains a plurality of bending struts (130) which are designed to fold out, during a transition from the insertion state to the anchoring state, by movement of a sleeve (405) arranged so as to be movable in the catheter relative to the crown (110), in order to allow the crown (110) to be unfurled, the bending struts (130) each having a first end which is fastened to the securing means,
**characterized in that**
the annular crown (110) consists of an unfurling element (120) which comprises a plurality of meander-shaped loops which are designed for unfurling during a transition of the device (100) from the insertion state to the anchoring state in order to increase the diameter of the crown (110),
the loops having a smaller distance from one another in the insertion state than in the anchoring state,
the connection means (115) containing a plurality of bending struts (130) arranged within the meander-shaped loops, each bending strut having a first end which is fastened to the securing means, and a second end which is connected to an end of the crown (110) remote from the securing means (105), and
the sleeve (405) arranged so as to be movable in the catheter relative to the crown (110) being designed to enclose the crown (110) in the insertion state of the device (100) for anchoring the ventricular assist system (205) and to release it to initiate the transition into the anchoring state.

4. Device according to claim 3, **characterized in that** the unfurling element (120) is formed from a shape memory material.

5. Device according to any of the preceding claims, **characterized by** an arc means (210) having at least one foot (215), the arc means (210) being designed to unfurl during the transition from the insertion state to the anchoring state in order to position the at least one foot (215) in the blood vessel, and the arc means (210) being connected to the crown (110) or the arc means (210) having an arc securing means (305) for securing the arc means (210) to the ventricular assist system (205).

6. Device according to claim 5, **characterized in that** the arc means (210) comprises three feet (215), in particular the feet (215) being formed for positioning the feet (215) in a cusp of a heart valve in each case.

7. Device according to any of the preceding claims, **characterized in that** the device (100) is cylindrical in the insertion state.

8. Device according to any of the preceding claims, **characterized in that** the crown (110) and the securing means (105) extend along a longitudinal axis which can correspond to the axis of a catheter, and the unfurling element (120) has an inclined position relative to this longitudinal axis of the device (100) in the anchoring state.

9. Device according to any of the preceding claims, **characterized in that** the fixing means (105) is designed to secure the device (100) to the ventricular assist system (205) in a form-fitting and/or force-fitting manner.

10. Arrangement comprising a ventricular assist system (205) which has a heart pump that can be inserted into a catheter and has a pump wheel and a suction tube, and comprising a device (100) designed according to any of claims 1 to 9.

11. Production method (2100) for producing a device (100) designed according to any of claims 1 to 10, the production method comprising at least the following steps:
providing (2101) a semi-finished product made of a shape memory material;
forming (2103) the securing means (105) and forming the crown (110) and forming the connection means (115) from the semi-finished product; and
heat-treating (2105) the securing means, the crown (110) and the connection means (115) in order to emboss the shape of the anchoring state.

## Revendications

1. Dispositif (100) permettant l'ancrage d'un système d'assistance cardiaque (205) dans un vaisseau sanguin, lequel dispositif peut occuper un état d'introduction pour l'introduction du système d'assistance cardiaque (205) dans le vaisseau sanguin et un état d'ancrage pour l'ancrage du système d'assistance cardiaque (205) dans le vaisseau sanguin et présente un appareil de fixation (105) pour la fixation au système d'assistance cardiaque (205) ainsi qu'une couronne (110) annulaire dont le diamètre est agrandi lors d'un passage du dispositif (100) de l'état d'introduction à l'état d'ancrage afin d'ancrer le système d'assistance cardiaque (205) dans le vaisseau sanguin, dans lequel le dispositif (100) permettant l'ancrage du système d'assistance cardiaque (205) comprend un appareil de liaison (115) qui est formé pour relier la couronne (110) à l'appareil de fixation (105) et qui contient plusieurs entretoises de flexion (130) qui sont conçues pour se déplier, par déplacement d'un manchon (405) disposé de manière mobile et déplaçable dans le cathéter par rapport à la couronne (110), lors d'un passage de l'état d'introduction à l'état d'ancrage afin de permettre un déploiement de la couronne (110), dans lequel les entretoises de flexion (130) comprennent respectivement une première extrémité qui est fixée à l'appareil de fixation,
**caractérisé en ce que**
la couronne (110) annulaire présente plusieurs éléments de déploiement (120) qui sont conçus pour permettre le déploiement lors d'un passage du dispositif (100) de l'état d'introduction à l'état d'ancrage pour l'agrandissement du diamètre de la couronne (110),
dans lequel chaque élément de déploiement (120) présente deux tiges de déploiement (125) reliées à ses extrémités,
dans lequel les tiges de déploiement (125) de chaque élément de déploiement (120) dans une section centrale située sur un chemin circulaire sont respectivement reliées à une tige de déploiement (125) d'un élément de déploiement (120) adjacent à l'élément de déploiement (120) dans une section centrale située sur un chemin circulaire,
dans lequel respectivement deux tiges de déploiement (125) adjacentes l'une à l'autre présentent l'une par rapport à l'autre un écartement plus réduit dans l'état d'introduction du dispositif (100) que dans l'état d'ancrage du dispositif (100), dans lequel les entretoises de flexion (130) présentent respectivement une seconde extrémité raccordée à un point de liaison de deux tiges de déploiement (125) adjacentes l'une à l'autre ou à une extrémité, opposée à l'appareil de fixation (105), de deux tiges de déploiement (125) de la couronne (110) reliées entre elles au niveau de ladite extrémité, et dans lequel
le manchon (405) disposé de manière mobile et déplaçable dans le cathéter par rapport à la couronne (110) est formé pour entourer la couronne (110) dans l'état d'introduction du dispositif (100) permettant l'ancrage du système d'assistance cardiaque (205), et pour la libérer afin d'induire le passage à l'état d'ancrage.

2. Dispositif selon la revendication 1, **caractérisé en ce que** les éléments de déploiement (120) sont formés à partir d'un matériau à mémoire de forme.

3. Dispositif (100) permettant l'ancrage d'un système d'assistance cardiaque (205) dans un vaisseau sanguin, lequel dispositif peut occuper un état d'introduction pour l'introduction du système d'assistance cardiaque (205) dans le vaisseau sanguin et un état d'ancrage pour l'ancrage du système d'assistance cardiaque (205) dans le vaisseau sanguin et présente un appareil de fixation (105) pour la fixation au système d'assistance cardiaque (205) ainsi qu'une couronne (110) annulaire dont le diamètre est agrandi lors d'un passage du dispositif (100) de l'état d'introduction à l'état d'ancrage afin d'ancrer le système d'assistance cardiaque (205) dans le vaisseau sanguin, dans lequel le dispositif (100) permettant l'ancrage du système d'assistance cardiaque (205) comprend un appareil de liaison (115) qui est formé pour relier la couronne (110) à l'appareil de fixation (105) et qui contient plusieurs entretoises de flexion (130) qui sont conçues pour se déplier, par déplacement d'un manchon (405) disposé de manière mobile et déplaçable dans le cathéter par rapport à la couronne (110), lors d'un passage de l'état d'introduction à l'état d'ancrage afin de permettre un déploiement de la couronne (110), dans lequel les entretoises de flexion (130) comprennent respectivement une première extrémité qui est fixée à l'appareil de fixation,
**caractérisé en ce que**
la couronne (110) annulaire est constituée d'un élément de déploiement (120) qui présente plusieurs boucles en forme de méandres, lesquelles boucles sont conçues pour permettre le déploiement lors d'un passage du dispositif (100) de l'état d'introduction à l'état d'ancrage pour l'agrandissement du diamètre de la couronne (110),
dans lequel les boucles dans l'état d'introduction présentent les unes par rapport aux autres un écartement plus réduit que dans l'état d'ancrage,
dans lequel l'appareil de liaison (115) contient plusieurs entretoises de flexion (130) disposées à l'intérieur des boucles en forme de méandres, lesquelles entretoises de flexion comprennent respectivement une première extrémité qui est fixée à l'appareil de fixation, et présentent une seconde extrémité qui est raccordée à une extrémité de la couronne (110), laquelle extrémité est opposée à l'appareil de fixation (105), et dans lequel
le manchon (405) disposé de manière mobile et déplaçable dans le cathéter par rapport à la couronne (110) est formé pour entourer la couronne (110) dans l'état d'introduction du dispositif (100) permettant l'ancrage du système d'assistance cardiaque (205), et pour la libérer afin d'induire le passage à l'état d'ancrage.

4. Dispositif selon la revendication 3, **caractérisé en ce que** l'élément de déploiement (120) est formé à partir d'un matériau à mémoire de forme.

5. Dispositif selon l'une des revendications précédentes, **caractérisé par** un appareil à arc (210) comportant au moins un petit pied (215), dans lequel l'appareil à arc (210) est conçu pour se déployer dans le vaisseau sanguin lors du passage de l'état d'introduction à l'état d'ancrage pour le positionnement de l'au moins un petit pied (215), et dans lequel l'appareil à arc (210) est relié à la couronne (110) ou dans lequel l'appareil à arc (210) présente un appareil de fixation d'arc (305) pour la fixation de l'appareil à arc (210) au système d'assistance cardiaque (205).

6. Dispositif selon la revendication 5, **caractérisé en ce que** l'appareil à arc (210) présente trois petits pieds (215), en particulier dans lequel les petits pieds (215) sont formés pour le positionnement des petits pieds (215) dans respectivement une cuspide d'une valve cardiaque.

7. Dispositif selon l'une des revendications précédentes,
**caractérisé en ce que** le dispositif (100) est cylindrique dans l'état d'introduction.

8. Dispositif selon l'une des revendications précédentes,
**caractérisé en ce que** la couronne (110) et l'appareil de fixation (105) s'étendent le long d'un axe longitudinal qui peut correspondre à l'axe d'un cathéter, et l'élément de déploiement (120) présente, dans l'état d'ancrage, une inclinaison par rapport audit axe longitudinal du dispositif (100).

9. Dispositif selon l'une des revendications précédentes,
**caractérisé en ce que** l'appareil de fixation (105) est formé pour fixer le dispositif (100) par complémentarité de forme et/ou à force au système d'assistance cardiaque (205).

10. Agencement comportant un système d'assistance cardiaque (205) qui présente une pompe cardiaque pouvant être introduite dans un cathéter, laquelle pompe comporte une roue de pompe et un tuyau d'aspiration, et comportant un dispositif (100) conçu selon l'une des revendications 1 à 9.

11. Procédé de fabrication (2100) pour la fabrication d'un dispositif (100) conçu selon l'une des revendications 1 à 10, dans lequel le procédé de fabrication présente au moins les étapes suivantes :
fourniture (2101) d'un produit semi-fini en un matériau à mémoire de forme ;
formation (2103) de l'appareil de fixation (105) et formation de la couronne (110) ainsi que formation de l'appareil de liaison (115) à partir du produit semi-fini ; et
traitement thermique (2105) de l'appareil de fixation, de la couronne (110) et de l'appareil de liaison (115) pour imprimer la forme de l'état d'ancrage.
